# EUROPÄISCHE PATENTSCHRIFT

(11) **EP 0 787 523 B1**
(45) Veröffentlichungstag und Bekanntmachung des Hinweises auf die Patenterteilung: **02.04.2003**
(21) Anmeldenummer: 97101249.7
(22) Anmeldetag: 28.01.1997
(51) Int. Cl.: B01D 61/00, B01D 69/14, A61M 1/14, G01N 33/543

(54) **Vorrichtung und Verfahren zur stoffspezifischen Behandlung von Fluiden**
Apparatus and process for material-specific treatment of fluids
Appareil et procédé pour le traitement spécifique à la matière des fluides

(30) Priorität: 01.02.1996 DE 19603523
(43) Veröffentlichungstag der Anmeldung: 06.08.1997
(73) Patentinhaber: MAT Adsorption Technologies GmbH & Co. KG, 63784 Obernburg (DE)
(72) Erfinder: Baurmeister, Ulrich, Dr., 42115 Wuppertal (DE); Wollbeck, Rudolf, 63906 Erlenbach (DE)
(74) Vertreter: Fett, Günter

(56) Entgegenhaltungen:
- EP-B- 0 112 094
- EP-B- 0 285 812
- WO-A-91/10492
- WO-A-92/18609

## Beschreibung

Die Erfindung betrifft eine Vorrichtung sowie ein Verfahren zur stoffspezifischen Behandlung eines Fluids durch Wechselwirkung mit in dem zu behandelnden Fluid enthaltenen Zielsubstanzen.

Unter Fluiden im Sinne der vorliegenden Erfindung werden Gase, Gasgemische, mit Partikeln beladene Gase sowie allgemein Flüssigkeiten wie z.B. klare Lösungen oder Suspensionen verstanden.

Stoffspezifische Behandlungen von Fluiden gewinnen in zunehmendem Maße an Bedeutung in Anwendungsgebieten wie der Biotechnologie, der Medizin oder der chemischen Technologie. Beispiele hierfür sind die Gewinnung von Wirkstoffen aus Zellsuspensionen, in der genmodifizierte Zellen Stoffe wie Antikörper, Hormone, Wachstumsfaktoren oder Enzyme in meist kleinen Konzentrationen produziert haben. Eine wichtige Anwendung ist auch die extrakorporeale Entfernung von unerwünschten Substanzen aus dem menschlichen Blut. Schließlich ist eine breite Anwendung auch die katalytische oder biokatalytische - enzymatische - Behandlung von Flüssigkeiten wie z.B. die Hydrolyse von Ölen durch Lipasen, die an einer Matrix immobilisiert sind. Bei vielen Anwendungen zur Behandlung von Flüssigkeiten enthalten diese Partikel verschiedenster Art, d.h. sie liegen als Suspensionen vor.

Die stoffspezifische Behandlung von Fluiden erfolgt vielfach derart, daß das zu behandelnde Fluid mit einem Trägermaterial in Kontakt gebracht wird, auf und/oder in dem wechselwirkende Gruppen oder Substanzen immobilisiert sind, die in spezifischer, selektiver Weise mit der in dem Fluid enthaltenen Zielsubstanz, d.h. der Substanz, auf die die stoffspezifische Behandlung ausgerichtet ist, wechselwirken. Solche Wechselwirkungen können beispielsweise Kationen- oder Anionenaustausch, Hydrophil-Hydrophob-Wechselwirkung, Wasserstoffbrückenbildung, Affinität oder enzymatische oder katalytische Reaktionen und dergleichen sein. Bei der affinen Stofftrennung sind an das Trägermaterial Liganden gekoppelt oder im Trägermaterial immobilisiert, die die Funktion haben, eine einzelne Zielsubstanz oder auch eine ganze Klasse von Substanzen adsorptiv spezifisch zu binden. Diese Zielsubstanz wird als Ligat bezeichnet. Ein Beispiel für klassenspezifische Liganden sind positiv geladene Diethylaminoethyl-(DEAE)-Gruppen oder negativ geladene Sulfonsäure(SO₃)-Gruppen, die die Klasse der positiv geladenen bzw. negativ geladenen Moleküle adsorbieren. Spezifische Liganden sind z.B. Antikörper gegen ein bestimmtes Protein, das als Ligat an den Antikörper gebunden wird.

Wesentliche Kriterien bei der stoffspezifischen Behandlung von Fluiden sind Produktivität und Selektivität. Mit Blick auf die Produktivität ist es wichtig, daß möglichst viele stoffspezifisch wirkende Gruppen pro Volumeneinheit zur Verfügung stehen, die mit der in dem zu behandelnden Fluid enthaltenen Zielsubstanz in Wechselwirkung treten können. Gleichzeitig ist eine Maximierung des Transports der Zielsubstanz zu den stoffspezifisch wirkenden Gruppen bzw. Substanzen anzustreben.

Ein in der Affinitätschromatographie häufig eingesetztes Trägermaterial für Liganden sind Sepharosepartikel, an die die Liganden gekoppelt sind und die in Form einer Schüttung in einer Chromatographiesäule vorliegen. Wenngleich sich hierbei eine hohe Konzentration an Liganden mit hoher Selektivität realisieren läßt, ist die Produktivität bekanntermaßen gering, da wegen der Kompressibilität der Sepharosepartikel der Durchsatz durch die Säule relativ gering bleiben muß. Darüberhinaus ist der Zugang der Ligaten zu den in den Sepharosepartikeln enthaltenen Liganden diffusionskontrolliert, wodurch insbesondere bei der Abtrennung von größeren Molekülen wie z.B. von Proteinen aufgrund deren geringen Diffusionsgeschwindigkeiten lange Verweilzeiten und damit nur geringe Durchsätze und geringe Produktivitäten resultieren.

Die US-A-4 202 775 offenbart ein aus porösen, starren Polymerpartikeln bestehendes Säulenmaterial, das als Adsorbents zur Abtrennung von organischen Komponenten eingesetzt werden kann, die an sich in wässriger Lösung befindlichen Proteinen adsorbiert sind. Dieses Säulenmaterial weist zwar nicht mehr den Nachteil der Kompressibilität auf, jedoch bleibt der Nachteil des diffusionskontrollierten Stofftransports in den Partikeln, verbunden mit langen Verweilzeiten und geringer Produktivität.

In der US-A-5 019 270 wird ein Chromatographie-Säulenmaterial aus starren, porösen Partikeln vorgestellt, bei dem ein Teilstrom der die Chromatographiesäule durchströmenden und zu behandelnden Flüssigkeit konvektiv diese Partikel durchströmt, dabei mit den in der porösen Struktur der Partikel befindlichen wechselwirkenden Gruppen in Kontakt kommt und anschließend wieder mit dem die Partikel umströmenden Flüssigkeitsstrom vereinigt wird. Aufgrund des konvektiven Stofftransports durch die Partikel ist gegenüber den zuvor genannten Säulenmaterialien eine Reduzierung der Verweilzeit und eine Steigerung der Produktivität möglich.

Zwar ist ein Vorteil von mit solchen Partikeln gefüllten Chromatographiesäulen, daß deren Aufbau und Verwendung sehr einfach ist. Jedoch ist allen partikulären Säulenmaterialien der Nachteil gemeinsam, daß bei Behandlung von Teilchen oder Zellen enthaltenden Flüssigkeiten, also von Suspensionen, die Partikel des Säulenmaterials relativ groß gewählt werden müssen, um eine gute Durchströmung der Partikelschüttung ohne Rückhalt von Teilchen oder z.B. Zellen der Flüssigkeit und ohne Tiefenfiltrationswirkung durch die Schüttung zu gewährleisten und um den Druckverlust gering zu halten. Hierdurch reduziert sich jedoch die pro Volumenelement zur Verfügung stehende Menge an stoffspezifisch wirkenden Gruppen, da das durch die Partikel in der Säule eingenommene Volumen kleiner wird. Darüberhinaus ist eine zunehmende Partikelgröße mit einer nachteiligen Verlängerung der Verweilzeiten verbunden.

Bei solchen Chromatographiesäulen ist anzustreben, daß die Partikel in einer geordneten Packung in der Säule vorliegen, um hierdurch den Anteil an Partikeln in der Schüttung zu maximieren und eine Vergleichmässigung der Strömung zwischen den Partikeln zu bewirken. Dies kann teilweise durch Verwendung von kugelförmigen Partikeln mit möglichst einheitlichem Durchmesser erreicht werden. Die Herstellung solcher einheitlicher Partikel ist jedoch aufwendig. Darüberhinaus läßt sich generell bei derartigen Partikelschüttungen nicht vermeiden, daß die Strömungskanäle zwischen den Partikeln unter fluiddynamischen Gesichtspunkten nicht optimal gestaltet sind. In den Zwickelbereichen zwischen den Partikeln kann es leicht zur Entstehung von Toträumen kommen, was insbesondere bei der stoffspezifischen Behandlung von Suspensionen zu negativen Erscheinungen wie z.B. dem Ablagern von suspendierten Teilchen in den Zwickelbereichen zwischen den Partikeln der Schüttung führen kann.

Die geschilderten Nachteile partikelförmiger Trägermaterialien führten zur Entwicklung einer Reihe von Verfahren zur stoffspezifischen Behandlung von Fluiden, bei denen Membranen mit poröser Struktur als Trägermaterialien für die wechselwirkenden Gruppen eingesetzt werden. Aufgrund ihrer porösen Struktur stellen diese Membranen eine große innere Oberfläche zur Verfügung, so daß an die Membranen in einer hohen Konzentration pro Volumeneinheit eine große Anzahl von funktionellen Gruppen gekoppelt werden kann, die in Wechselwirkung mit den die Membran durchströmenden, zu behandelnden Fluiden treten (s. z.B. E. Klein, "Affinity Membranes", John Wiley & Sons, Inc., 1991; S. Brandt u. a., "Membrane-Based Affinity Technology for Commercial Scale Purifications", Bio/Technology Vol. 6 (1988), S. 779-782).

Über die Ausführung der verwendeten Membran kann eine Anpassung an die Erfordernisse des Behandlungsverfahrens erfolgen. Es stehen Membranen in Form von Hohlfasern oder als Flachmembranen aus unterschiedlichsten Materialien zur Verfügung, so daß eine Anpassung an die physikochemischen Eigenschaften der zu behandelnden Fluide möglich ist. Auch die Porengröße der Membranen kann so eingestellt werden, daß das zu behandelnde Fluid mit der in ihm enthaltenen Zielsubstanz durch die Membran konvektiv hindurchströmen kann und - im Falle der Anbindung der Zielsubstanz an die wechselwirkenden Gruppen - keine Blockierung der Membran eintritt.

Durch die Dicke der Membranwand läßt sich die Verweilzeit des zu behandelnden Fluids in der Membran sowie der bei der Durchströmung entstehende Druckverlust beeinflussen. Hierbei zeichnen sich Membranen aufgrund ihrer in der Regel nur geringen Wanddicke (z.B. <100 µm) durch kurze Transportwege des zu behandelnden Fluids zu den wechselwirkenden Gruppen aus, wodurch die Verweilzeiten vergleichsweise kurz sind. Gleichzeitig ergibt sich als weiterer Vorteil bei der Verwendung von Membranen als Trägermaterial gegenüber partikelförmigen Trägermaterialien, daß sich aufgrund der im wesentlichen gleichförmigen Dicke der Membranwand eine gleichmäßigere Durchströmung des Trägermaterials und daraus folgend eine engere Verweilzeitverteilung sowie gleichmäßigere und vollständigere "Nutzung" der wechselwirkenden Gruppen resultiert.

Es sind eine Reihe von solche Membranen enthaltenden Vorrichtungen beschrieben, die bei Verfahren zur stoffspezifischen Behandlung von Fluiden verwendet werden und bei denen sowohl Flachmembranen als auch Hohlfasermembranen zum Einsatz kommen. Dabei ist zwischen der sogenannten dead-end-Filtration bzw. den dead-end-Modulen und der cross-flow-Filtration bzw. den cross-flow-Modulen zu unterscheiden.

Bei der dead-end-Filtration wird das gesamte, als Feedstrom in den Membranmodul einströmende Fluid durch die Membran hindurchgeführt und auf der der Einströmseite der Membran gegenüberliegenden Abströmseite als Filtrat bzw. Permeat abgeleitet.

Bei der Fahrweise im cross-flow-Modus fließt der Feedstrom parallel zur einen Seite der Membran. Dabei tritt ein Teil des Feedstroms durch die Membran hindurch. Der hindurchgetretene Teilstrom wird als Permeat, der auf der Feedstromseite verbleibende Teilstrom als Retentat abgeleitet. Hierbei kann auch auf der Permeatseite der Membran ein zusätzlicher Fluidstrom eingeleitet werden, der den durch die Membran hindurchgetretenen Teilstrom aufnimmt.

In der US-A-4 935 142 wird eine Vorrichtung zur Durchführung von Affinitätstrennverfahren im dead-end-Modus beschrieben, die Stapel aus Flachmembranen enthält. An die Flachmembranen sind Liganden gekoppelt, an die die aus der zu behandelnden Flüssigkeit abzutrennenden Ligaten gebunden werden. Die den Membranstapel aufbauenden Flachmembranen sind gegen das umgebende Gehäuse abgedichtet, so daß eine Zwangsströmung durch den Membranstapel erzeugt wird. Nachteilig bei einem derartigen Aufbau ist der hohe Druckverlust bei der Durchströmung des Stapels, wobei auch zusätzliche Maßnahmen erforderlich sind, den Flachmembranelementen eine hinreichende Stabilität gegenüber den auftretenden hohen Drücken zu verleihen.

In der EP-A-0 173 500, der EP-A-0 280 840 und der EP-A-0 610 755 werden ebenfalls Vorrichtungen für den Einsatz bei membranbasierten Affinitätstrennverfahren wie z. B. der Isolierung von Immunglobulinen, Antigenen u.ä. beschrieben. Diese Vorrichtungen bzw. Membranmodule enthalten sternförmig gefaltete, mikroporöse Flachmembranen. Die sternförmig gefalteten Flachmembranen sind zwischen zwei groben Gittern abgestützt und zwischen zwei koaxial zueinander angeordneten zylinderförmigen Gehäuseelementen eingebracht. Bevorzugt können auch mehrere sternförmig gefalteten Flachmembranen konzentrisch zueinander im Gehäuse angeordnet sein. Ähnlich aufgebaute Module werden in der EP-A-0 662 340 beschrieben, wobei in die gefaltete Flachmembranstruktur kleine Partikel mit spezifisch wechselwirkenden Gruppen eingelagert sind.

Die zu behandelnde Flüssigkeit wird bei den genannten Vorrichtungen unter Druckbeaufschlagung von innen nach außen oder auch in umgekehrter Richtung durch den Modul geführt und durchströmt dabei im dead-end-Modus konvektiv die Membran. Gegenüber Modulen mit ungefalteten, konzentrisch zueinander angeordneten Membranen bieten die genannten Module den Vorteil einer relativ größeren Membranfläche bei vergleichsweise geringerem Druckverlust. Jedoch sind in der Regel nur geringe Füllgrade, definiert als Membranvolumen bezogen auf das Gesamtvolumen des Moduls, möglich.

Bei allen im dead-end-Modus zu betreibenden Membranmodulen ist von Nachteil, daß sie für die Behandlung von Teilchen enthaltenden Fluiden, d.h. z.B. von Suspensionen, dann nicht geeignet sind, wenn die im Fluid enthaltenen Teilchen in der Größenordnung der Porendurchmesser liegen. Die Teilchen würden zum Aufbau einer Schicht auf der Membranwand führen und die Membran blockieren. Für die Anwendung bei z.B. Affinitätstrennverfahren für teilchenenthaltende Flüssigkeiten, d.h. von Suspensionen können derartige dead-end-Module nur in Kombination mit einer vorgeschalteten Vorfiltrations-/Vorreinigungsstufe betrieben werden. Hierdurch verliert ein solches Verfahren jedoch an Effizienz, etwa auch dadurch, daß in vielen Fällen durch eine solche Vorreinigung ein Großteil der Zielsubstanz verloren geht.

Die genannten Nachteile im dead-end-Modus betriebener Module in Bezug z.B. auf ihre Verwendbarkeit bei Suspensionen können zumindest teilweise bei Einsatz von cross-flow-Modulen vermieden werden. Bei diesen läßt sich durch den parallel zur Membranoberfläche strömenden Feedstrom bei genügend hohen Scherbeanspruchungen der Aufbau einer Schicht aus suspendierten Teilchen verringern.

Die WO 90/05018 offenbart einen Membranmodul zur Anwendung bei Affinitätstrennverfahren, der vom Aufbau einem cross-flow-Modul entspricht. Eine ligathaltige Flüssigkeit wird über eine Einlaßvorrichtung in das Modulgehäuse eingeleitet und strömt tangential über die eine Seite einer beispielsweise hohlfaserförmigen Membran, an die Liganden gekoppelt sind. Ein Teil der Flüssigkeit tritt in die Membran ein, strömt durch diese hindurch, wobei die Ligaten an die Liganden angelagert werden, und tritt auf der der Eintrittsseite gegenüberliegenden Membranseite als Permaetstrom aus. Über getrennte Auslaßeinrichtungen werden Retentatstrom und Permeatstrom abgeleitet. Wesentliches Merkmal der gemäß WO 90/05018 verwendeten Membranen ist eine isotrope, mikroporöse Struktur, die eine Konvektionsströmung von Makromolekülen enthaltenden Lösungen ermöglicht.

In der US-A-4 266 026 wird ein cross-flow-Modul, der anisotrope Hohlfasermembranen enthält, für ein Verfahren zur Durchführung katalytischer Reaktionen beschrieben. Bei den hierbei eingesetzten Katalysatoren handelt es sich insbesondere um Enzyme, die in der Membranstruktur beispielsweise über Kopplungs-Reagenzien immobilisiert sind. Die zu behandelnde Flüssigkeit fließt als Feedstrom unter Druck durch das Lumen der Hohlfasern. Ein Teil der Flüssigkeit durchströmt dabei konvektiv die Membranwand und wird dabei der katalytischen Reaktion unterworfen. Als Beispiel wird die katalytische Umsetzung von Laktose in Glukose und Galaktose mittels Galaktosidase als Katalysator ausgeführt. Retentat und Permeat werden als getrennte Flüssigkeitsströme aus dem Modul abgeführt, in einem Vorratsbehälter miteinander vereinigt und lumenseitig solange in den Modul im Kreislauf zurückgeführt, bis der gewünschte Umsatz der reagierenden Substanz erreicht ist.

Eine Abwandlung eines cross-flow-Prozesses wird in der WO 93/02777 beschrieben. Zur spezifischen Entfernung bestimmter Komponenten aus dem Blut dient ein U-förmig in ein speziell ausgeformtes Gehäuse eingebettetes Bündel aus semipermeablen Hohlfasermembranen, das als Plasmafilter fungiert. Die Hohlfasermembranen werden lumenseitig vom Blut durchströmt, die stoffspezifische Behandlung erfolgt an dem mittels der Membran abgetrennten Blutplasma im Außenraum um die Hohlfasermembranen. In diesem Außenraum befindet sich zur Anlagerung der abzutrennenden Komponenten ein z.B. immobilisierte Enzyme oder Antikörper enthaltendes Reinigungsmedium. Das Bündel läßt sich prinzipiell in einen Einströmast und in einen Ausströmast unterteilen. Aufgrund des im Bereich des Einströmasts auftretenden positiven Transmembrandrucks erfolgt ein der Diffusion überlagerter konvektiver Transport von Blutplasma durch die Membran in den Außenraum. Im Bereich des Ausströmasts strömt das behandelte Plasma aufgrund des dort auftretenden negativen Transmembrandrucks in das Lumen der Hohlfasermembranen zurück und wird wieder mit dem Blut vereinigt.

Das Verfahren gemäß der WO 93/02777 bietet den Vorteil, daß keine separaten Pumpen und/oder Kontrollorgane für den Permeatstrom, d.h. den Plasmastrom benötigt werden. Jedoch besitzen die verwendeten Module ein großes Totraumvolumen im Außenbereich der Membranen.

Bei der Vorrichtung zur Blutbehandlung gemäß EP-A-0 112 094 wird ebenfalls das Permeat nicht separat aus dem eingesetzten Modul-herausgeführt, sondern innerhalb des Moduls wieder mit dem Retentat, d.h. in diesem Fall dem Blut zusammengeführt. Auch hier dient die Membran als Plasmaseparator, wobei von der einen Seite der Membran ein Kanal ausgebildet wird, durch den das Blut strömt, und von der anderen Membranseite und dem umgebenden Gehäuse ein Behandlungsraum, in dem sich ein Material zur stoffspezifischen Behandlung befindet. Durch eine geeignete Vorrichtung werden auf den Behandlungsraum Druckvariationen aufgeprägt. Hierdurch wird die Druckdifferenz zwischen Blutkanal und Behandlungsraum derart periodisch verändert, daß im Wechsel zunächst Plasma aus dem Blut durch die Membran in den Behandlungsraum permeiert, wo es der stoffspezifischen Behandlung unterzogen wird. Anschließend strömt das behandelte Plasma in umgekehrter Richtung wieder durch die Membran und wird mit dem Blut vereinigt.

Ein ähnliches Prinzip wird in der WO 80/02805 verfolgt. Auch gemäß dieser Schrift wird durch Druckoszillationen ein Teil des Feedstroms konvektiv durch die Membran und wieder zurück in den Feedstrom geführt. Im Unterschied zu EP-A-0 112 094 ist bei der Vorrichtung gemäß der WO 80/02805 das biologisch aktive Material, an das die Zielsubstanz der zu behandelnden Flüssigkeit gebunden werden soll, in den Poren und an der Oberfläche der Membran an die Membran gekoppelt, so daß die stoffspezifische Behandlung der Flüssigkeit bei der Durchströmung der Membran stattfindet.

In der EP-A-0 341 413 wird ein Adsorbermodul zur Behandlung von Vollblut beschrieben, bei dem die im Modul enthaltenen, mit Liganden versehenen Hohlfasermembranen im cross-flow-Modus lumenseitig vom Blut durchströmt werden. Hierbei tritt Plasma als Permeat durch die Hohlfasermembranwand in den die Hohlfasermembranen umschließenden Außenraum, wobei in der Membranwand die Behandlung des Plasmas erfolgt. In einer speziellen Ausführungsform besitzt dieser Modul keinen Auslaß für das Permeat, sondern bei der Behandlung von Vollblut sammelt sich das als Permeat abgetrennte Plasma im Außenraum um die Kapillaren und tritt infolge der sich einstellenden Druckverhältnisse wieder durch die Hohlfasermembranwandung in das Lumen der Hohlfasermembran ein. Ein solches Modulkonzept hat jedoch den Nachteil, daß auf den die Membran durchtretenden Plasmastrom nur in eingeschränktem Maße Einfluß genommen werden kann. Darüberhinaus sind die erforderlichen Behandlungszeiten relativ lang, da allein zur Befüllung des Außenraums um die Hohlfasermembranen herum Permeatzeiten von mehr als 10 min. erforderlich sind.

Die beschriebenen, in cross-flow-Modus betriebenen Module zur stoffspezifischen Behandlung von Flüssigkeiten weisen Nachteile wie z.B. das Erfordernis zusätzlicher Pumpen und/oder Kontrollorgane aufgrund getrennter Permeat- und Retentatströme oder zusätzlicher Aggregate zur Erzeugung von Druckoszillationen auf. Allen gemeinsam ist der Nachteil, daß stets eine Einbettung der Membranen in das Gehäuse erforderlich ist, wodurch die Herstellung der Membranmodule aufwendig wird. Dies wirkt sich insbesondere dann nachteilig aus, wenn der Behandlungsprozeß die Hintereinanderschaltung mehrerer Module erforderlich macht. Ferner ist dies von Bedeutung, wenn zur Kopplung oder Polymerisation der Liganden aggressive Lösemittel wie z.B. Toluol zum Einsatz kommen und diese Kopplung in der Vorrichtung stattfindet. In der Praxis hat sich gezeigt, daß eine dann erforderliche lösungsmittelbeständige Einbettung sehr aufwendig und mit hohen Kosten verbunden ist.

Es ist daher Aufgabe der Erfindung, eine Vorrichtung der eingangs genannten Art zur stoffspezifischen Behandlung von Fluiden zur Verfügung zu stellen, bei der die genannten Nachteile des Standes der Technik zumindest reduziert sind, die in einfacher Weise herstellbar ist, die flexibel an die jeweilige Fluidbehandlung angepaßt werden kann und die auch für klare Lösungen und insbesondere für Suspensionen geeignet ist.

Es ist des weiteren Aufgabe der Erfindung, ein Verfahren der eingangs genannten Art zur effizienten stoffspezifischen Behandlung von Fluiden unter Verwendung von semipermeablen Membranen mit poröser Struktur bereitzustellen, das auch für die Behandlung von Suspensionen geeignet ist.

Die Aufgabe wird durch eine Vorrichtung zur stoffspezifischen Behandlung eines Fluids durch Wechselwirkung mit in dem zu behandelnden Fluid enthaltenen Zielsubstanzen gelöst, wobei die Vorrichtung besteht aus
a) einem Gehäuse,
b) einer Einlaßeinrichtung zum Einleiten des zu behandelnden Fluids in das Gehäuse,
c) einer Auslaßeinrichtung zum Ableiten des behandelten Fluids aus dem Gehäuse,
d) mindestens einem Behandlungselement zur stoffspezifischen Behandlung des Fluids mit einem der Einlaßeinrichtung und einem der Auslaßeinrichtung zugewandten Ende und einer Wand, die zumindest teilweise aus mindestens einer semipermeablen Membran mit einer porösen Struktur gebildet ist,
wobei das mindestens eine Behandlungselement mindestens einen durch die Wand des Behandlungselements gebildeten Hohlraum aufweist, wobei der Hohlraum geschlossen oder höchstens in Richtung der Auslaßeinrichtung einseitig geöffnet ist, wobei das mindestens eine Behandlungselement derart im Gehäuse angeordnet ist, daß zwischen Einlaßeinrichtung und Auslaßeinrichtung ein durchgehendes, vom zu behandelnden Fluid durchströmbares, das mindestens eine Behandlungselement berührendes und umgebendes und das mindestens eine Behandlungselement zumindestens an seinem der Einlaßeinrichtung und an seinem der Auslaßeinrichtung zugewandten Ende im wesentlichen umschließendes Kanalsystem ausgebildet ist und wobei auf und/oder in der Membran stoffspezifisch wirkende Gruppen immobilisiert sind.

Die Aufgabe wird desweiteren durch ein Verfahren zur stoffspezifischen Behandlung eines Fluids durch Wechselwirkung mit in dem zu behandelnden Fluid enthaltenen Zielsubstanzen unter Verwendung dieser Vorrichtung gelöst, wobei die Vorrichtung mindestens ein Behandlungselement enthält, welches zumindest teilweise aus mindestens einer semipermeablen Membran mit poröser Struktur ausgebildet ist, wobei die Membran mindestens eine erste, die Außenseite definierende Oberfläche sowie mindestens eine zweite, die Innenseite definierende Oberfläche aufweist, und wobei das Verfahren mindestens die Schritte umfasst:
a) Einleiten des zu behandelnden Fluids in das Gehäuse,
b) Durchströmen des Gehäuses mit besagtem Fluid, dabei Vorbeileiten des zu behandelnden Fluids als Primärstrom an der Außenseite der Membran, nicht jedoch an ihrer Innenseite derart, daß ein Teil dieses Primärstroms als Sekundärstrom über die Außenseite in die Membran einströmt, durch die Membran hindurchströmt, wobei an dem den Sekundärstrom bildenden Teil des zu behandelnden Fluids die stoffspezifische Behandlung des Fluids erfolgt, und anschließend durch die Innenseite aus der Membran herausströmt,
c) Ausleiten des behandelten Fluids aus dem Gehäuse,
wobei die Membran über ihre gesamte Erstreckung an ihrer Außenseite im wesentlichen frei vom Primärstrom umströmt wird und daß der durch die Membran hindurchgeströmte Sekundärstrom nach der stoffspezifischen Behandlung dem an der Außenseite der Membran strömenden Primärstrom wieder zugeführt wird.

Unter zu behandelnden Fluiden sind im Rahmen der vorliegenden Erfindung solche Fluide zu verstehen, die bestimmte Stoffe oder Zielsubstanzen enthalten, auf die die stoffspezifische Behandlung ausgerichtet ist.

Die erfindungsgemäße Vorrichtung ist so aufgebaut, daß das zu behandelnde Fluid über die Einlaßeinrichtung in das Gehäuse eingebracht wird und als Primärstrom im Gehäuse durch das Kanalsystem in Richtung der Auslaßeinrichtung an dem im Gehäuse angeordneten mindestens einen Behandlungselement vorbeiströmt. Hierbei ist das mindestens eine Behandlungselement in das Gehäuse der erfindungsgemäßen Vorrichtung eingebracht, ohne daß eine Einbettung der Enden der mindestens einen Behandlungselements erforderlich ist, d.h. das der Einlaßeinrichtung und das der Auslaßeinrichtung zugewandte Ende sind frei von einer das jeweilige Ende umschließenden Einbettung. Auf diese Weise sind auch die Enden des mindestens einen Behandlungselements im wesentlichen vom Primärstrom umströmbar.

Aufgrund des durch die Strömung im Kanalsystem erzeugten Druckgefälles entlang des Behandlungselements dringt ein Teil des Primärstroms als Sekundärstrom in das Behandlungselement ein und durchströmt die semipermeable Wand des Behandlungselements, wobei die stoffspezifische Behandlung in Bezug auf die Zielsubstanz erfolgt, und sammelt sich in dem mindestens einen Hohlraum des Behandlungselements.

Die Höhlräume können geschlossen oder höchstens in Richtung der Auslaßeinrichtung geöffnet sein. Überraschenderweise hat sich gezeigt, daß sich auch bei geschlossenen Hohlräumen ein Sekundärstrom durch die solche Hohlräume aufweisenden Behandlungselemente ausbildet. Bevorzugt wird jedoch eine Ausführungsform der erfindungsgemäßen Vorrichtung, bei der mindestens ein Hohlraum in Richtung der Auslaßöffnung eine Öffnung aufweist, die in das Kanalsystem mündet.

Für den Fall des geschlossenen Hohlraums tritt der Sekundärstrom aufgrund des Druckgefälles im oberen, der Einlaßeinrichtung zugewandten Abschnitt des Behandlungselements durch die semipermeable Wand in das Element ein, sammelt sich im Hohlraum und tritt im unteren, der Auslaßeinrichtung zugewandten Abschnitt des Behandlungselements aufgrund des verringerten Drucks in der Primärströmung in umgekehrter Richtung aus dem Hohlraum durch die semipermeable Wand in das Kanalsystem aus, wo er wieder mit dem Primärstrom vereinigt wird. Hierbei kann die stoffspezifische Behandlung der den Teilstrom bildenden Fluid sowohl beim ersten als auch beim zweiten Durchgang durch die Wand erfolgen.

Für den bevorzugten Fall, daß mindestens ein Hohlraum in Richtung der Auslaßeinrichtung eine Öffnung aufweist, die in das Kanalsystem mündet, tritt der Sekundärstrom im wesentlichen über die gesamte Erstreckung des Behandlungselements zwischen Einlaßeinrichtung und Auslaßeinrichtung in das Behandlungselement ein mit einem abnehmenden Gradienten des Stoffstromes in Richtung auf die Auslaßvorrichtung. Der gesamte durch die semipermeable, poröse Wand hindurchtretende Sekundärstrom sammelt sich im Hohlraum und strömt am unteren Ende des Elements durch die Öffnung in das Kanalsystem zurück, wo er wieder mit dem Primärstrom vereinigt wird. Diese Ausführung des in Richtung auf die Auslaßeinrichtung geöffneten und mit dem Kanalsystem in Verbindung stehenden Hohlraums bietet den Vorteil, daß sich gegenüber einem geschlossenen Hohlraum größere Sekundärströme, d.h. höhere Durchflüsse durch die Wand eines Behandlungselements einstellen, da der Sekundärstrom beim Austritt aus dem Behandlungselement nicht den durch die semipermeable Wand des Behandlungselements aufgebauten Strömungswiderstand überwinden muß.

Das Erfordernis des höchstens in Richtung der Auslaßeinrichtung geöffneten Hohlraums resultiert aus der Anwendbarkeit der erfindungsgemäßen Vorrichtung für Suspensionen gemäß der Aufgabenstellung. Beispielsweise würde ein Behandlungselement mit in Richtung auf die Einlaßeinrichtung geöffnetem Hohlraum wie ein dead-end-Filter wirken, was bei einer Behandlung von Suspensionen zur Folge hätte, daß sich die suspendierten Teilchen im Hohlraum absetzen würden.

Bei der Dimensionierung des Hohlraums kommt es darauf an, daß der Strömungswiderstand, der bei der Durchströmung des Hohlraums durch den Sekundärstrom entsteht, klein gegenüber dem bei der Durchströmung der semipermeablen Wand entstehenden Strömungswiderstand ist. Gleichzeitig ist anzustreben, daß pro Behandlungselement ein möglichst hoher Anteil des Volumens des Behandlungselements aus semipermeabler poröser Membranwand besteht, in der die stoffspezifische Behandlung erfolgt. Ein bevorzugtes Verhältnis V_{w}/V_{b} des Volumens der Wände eines Behandlungselements V_{w}, bezogen auf das aus dem Volumen der Wände V_{w} und dem Volumen des mindestens einen Hohlraums Vₕ zusammengesetzten Volumen V_{b} des Behandlungselements liegt im Bereich 0,5 < V_{w}/V_{b} < 0,98, ein besonders bevorzugtes Verhältnis liegt im Bereich 0,6 < V_{w}/V_{b} < 0,85.

Neben anderen Einflußfaktoren wird der durch ein Behandlungselement strömende Sekundärstrom durch den längs des Behandlungselements bestehenden Druckgradienten dp/dx bestimmt. Dabei ist dp die differentielle Druckänderung längs einer differentiellen Strecke dx in Richtung des Primärstroms. Je größer dieser Druckgradient ist, umso größer ist der Sekundärstrom durch das Behandlungselement und umso größer ist demzufolge der Teil des Primärstroms, der der stoffspezifischen Behandlung unterzogen wird. Das Druckgefälle entlang eines Behandlungselements bzw. entlang des das Behandlungselement umgebenden Kanalsystems nimmt mit steigendem Durchsatz durch das Kanalsystem, d.h. mit steigendem Primärstrom zu.

Das Druckgefälle dp/dx nimmt zu, je größer das als Füllgrad bezeichnete Verhältnis V_{B}/V_{G} ist, wobei V_{B} das aus der Summe der Volumina V_{b} der einzelnen Behandlungselemente zusammengesetzte Gesamtvolumen aller Behandlungselemente und V_{G} das Volumen des leeren Gehäuses ist. Bewährt hat sich ein Verhältnis V_{B}/V_{G} zwischen 0,4 und 0,95, bestens bewährt ein Verhältnis zwischen 0,55 und 0,75. Bei derartigen Füllgraden werden nämlich die Abstände zwischen den Behandlungselementen und somit die entsprechenden Dimensionen der Querschnitte der Kanäle des Kanalsystems so klein, daß in Verbindung mit geeigneten Durchsätzen durch bzw. Strömungsgeschwindigkeiten im Kanalsystem genügend hohe Druckgradienten entstehen, um dem diffusiven Transport durch die poröse Membranstruktur einen wesentlich größeren konvektiven Transport aufgrund der Sekundärströmung zu überlagern.

Es ist zweckmäßig, daß das Behandlungselement und sein mindestens einer Hohlraum ihre Längserstreckung in Richtung der Durchströmung des Gehäuses haben. Von Vorteil ist ein Abmessungsverhältnis L/D des mindestens einen Hohlraums zwischen 2 und 4000, wobei L die Hohlraumabmessung in Durchströmrichtung des Gehäuses und D der hydraulische Durchmesser des Hohlraumquerschnitts senkrecht dazu ist. Der hydraulische Durchmesser D ist dabei definiert durch die Beziehung D = 4*A/U mit A als der Fläche besagten Querschnitts und U als seinem Umfang. Besonders vorteilhaft sind Abmessungsverhältnisse L/D zwischen 20 und 450.

Hierbei werden Behandlungselemente bevorzugt, deren Wände in ihrer Ausdehnung senkrecht zur Richtung der Primärströmung eine im wesentlichen gleichmäßige Dicke aufweisen. Dadurch wird auch erreicht, daß der Sekundärstrom bei der Durchströmung der Membran an jeder Stelle eines Behandlungselements im wesentlichen gleiche Durchströmwege zurücklegt, was im Hinblick auf eine möglichst gleichmäßige Verweilzeit des zu behandelnden Fluids von besonderem Vorteil ist.

Im Falle der Anordnung eines einzelnen Behandlungselements im Gehäusequerschnitt ist es erforderlich, das Kanalsystem zwischen Behandlungselement und Gehäuseinnenwand auszubilden, etwa durch separate Abstandshalter oder eine entsprechende Formgebung des Behandlungselements und/oder der Gehäuseinnenwand, wobei in Hinblick auf eine möglichst gleichmäßige Umströmung des Behandlungselements das Kanalsystem ebenfalls möglichst gleichförmig ausgebildet sein sollte.

Im Hinblick auf die Behandlung größerer Fluidmengen ist es jedoch vielfach zweckmäßig, Behandlungselemente zu mindestens einer Gruppe von mindestens zwei, vorzugsweise aber mehreren Behandlungselementen zusammenzufassen, wobei die Behandlungselemente im wesentlichen quer zur Richtung der Erstreckung des Kanalsystems zwischen Einlaßeinrichtung und Auslaßeinrichtung nebeneinander angeordnet sind.

Hierbei ist es von Vorteil, wenn die zu einer Gruppe zusammengefaßten Behandlungselemente durch Abstandshalter gegeneinander auf Abstand gehalten werden, so daß sich um die Behandlungselemente herum ein Kanalsystem mit definierten Kanälen ausbildet, durch das eine gleichmäßige Durchströmung der Gruppe aus Behandlungselementen und eine gleichmäßige Umströmung der einzelnen Behandlungselemente ermöglicht wird. Darüberhinaus läßt sich über die Abstandshalter die Größe der Strömungsquerschnitte der Kanäle einstellen, wodurch im Falle der stoffspezifischen Behandlung von Suspensionen eine Anpassung an die Größe der in der Suspension enthaltenen Teilchen ermöglicht wird und dadurch einer Verstopfung des Kanalsystems vorgebeugt werden kann. Gleichzeitig kann über die Einstellung der Strömungsquerschnitte Einfluß auf den sich in den Kanälen einstellenden Druckgradienten genommen werden.

Zweckmäßigerweise sind die Abstandshalter so ausgeführt, daß sie eine elastische Komponente aufweisen. Hierdurch kann eine solche Gruppe aus Behandlungselementen auf einfache Weise in das Gehäuse der Vorrichtung eingebracht werden, indem die Gruppe zunächst geringfügig zusammengedrückt wird, wobei der Abstand zwischen den Behandlungselementen etwas verringert wird, in das Gehäuse eingeschoben wird und anschließend entspannt wird, wodurch sich der Abstand zwischen den Behandlungselementen wieder vergrößert. Als Folge wird erreicht, daß die Gruppe von Behandlungselementen an ihrem äußeren Umfang an der Gehäuseinnenwand anliegt, und eine unerwünschte Randströmung entlang der Gehäuseinnenwand wird zumindest reduziert. Bevorzugt wird ein Abstand zwischen den an die Gehäusewand angrenzenden Behandlungselementen und der Gehäuseinnenwand, der kleiner oder gleich dem Abstand zwischen den Behandlungselementen ist.

Die Abstandshalter können auch als dünne Röhrchen oder Kapillaren ausgebildet sein, deren Achse im wesentlichen parallel zur Erstreckung der Behandlungselemente einer Gruppe zwischen Einlaßeinrichtung und Auslaßeinrichtung liegt und die in ihrem Lumen von Primärstrom durchströmt werden können. Vorzugsweise sind diese Röhrchen bzw. Kapillaren nicht entlang der gesamten Erstreckung der Behandlungselemente zwischen Einlaßeinrichtung und Auslaßeinrichtung angeordnet, sondern in einem Teilbereich dieser Erstreckung an dem der Auslaßeinrichtung zugewandten Ende der Behandlungselemente. Auf diese Weise läßt sich der Druckabfall des Primärstroms erhöhen und damit der Sekundärstrom durch die Behandlungselemente vergrößern.

Ähnliche Effekte lassen sich auch durch eine entsprechende Ausgestaltung des Gehäusequerschnitts erzielen, wenn sich der Gehäusequerschnitt im Bereich einer Stufe von Behandlungselementen jeweils zur Auslaßeinrichtung hin verjüngt. Hierdurch wird der Abstand zwischen den Behandlungselementen an deren der Auslaßeinrichtung zugewandten Enden gegenüber dem Abstand an deren der Einlaßeinrichtung zugewandten Enden reduziert.

Zur Erhöhung des Wirkungsgrades der stoffspezifischen Behandlung ist es von Vorteil, wenn im Gehäuse in Richtung der Erstreckung-des Kanalsystems zwischen Einlaßeinrichtung und Auslaßeinrichtung, d.h. in Richtung der Durchströmung des Gehäuses mehrere Behandlungselemente oder Gruppen von Behandlungselementen als Stufen hintereinander angeordnet sind. Durch die Hintereinanderschaltung einer Vielzahl von Stufen kann gleichzeitig die einzelne Stufe in ihrer Abmessung in Richtung der Durchströmung des Gehäuses kurz gehalten und dadurch eine Konzentrationsänderung bezüglich möglicher kritischer Komponenten in dem zu behandelnden Fluid vermieden werden. Dies ist insbesondere bei solchen Verfahren zur stoffspezifischen Behandlung von Suspensionen von Bedeutung, bei denen zumindest ein Teil der suspendierten Teilchen durch die semipermeable Membranwand der Behandlungselemente zurückgehalten werden soll und gleichzeitig zu hohe Aufkonzentrationen an suspendierten Teilchen vermieden werden sollen, wie z.B. bei der stoffspezifischen Behandlung von Blut. Durch vergleichsweise kurze Behandlungselemente wird entlang der Behandlungselemente dem Primärstrom nur ein geringer Teilstrom entzogen, so daß die Konzentrationsänderungen bis zur folgenden Zusammenführung von Primär- und Sekundärstrom gering bleiben.

Bevorzugt liegt bei der erfindungsgemäßen Vorrichtung die Anzahl der Stufen im Gehäuse zwischen 1 und 1000. Bewährt hat sich eine Stufenzahl zwischen 1 und 100, bestens bewährt eine Stufenzahl zwischen 1 und 10. Dabei ist es vorteilhaft, wenn die einzelnen Stufen einen Abstand zueinander aufweisen, um weder den Primärstrom noch den Sekundärstrom lokal durch die in Durchströmungsrichtung des Gehäuses nachfolgend angeordenete Stufe zu behindern und um eine gute Durchmischung von Primärstrom und Sekundärstrom zu ermöglichen. Eine gleichmäßige Durchmischung ist von Vorteil, um z.B. unerwünschte Konzentrationsschwankungen zu vermeiden. Bevorzugt liegt das Verhältnis s/D des Abstands s zwischen zwei aufeinanderfolgenden Stufen zum hydraulischen Durchmesser D der Hohlräume der Behandlungselemente zwischen 0 und 5.

Natürlich ist in Anpassung an die Erfordernisse des Behandlungsverfahrens auch die Hintereinanderschaltung mehrerer erfindungsgemäßer Vorrichtungen möglich, die vorzugsweise mehrere Stufen von Behandlungselementen enthalten.

In der bevorzugten Ausführung der Erfindung strömt der Sekundärstrom durch die semipermeable Wand der Behandlungselemente und transportiert konvektiv die Zielsubstanz durch die Membran. Dies setzt voraus, daß die zur Ausbildung der Behandlungselemente-eingesetzte semipermeable Membran mit poröser Struktur eine Porengröße aufweist, die einen konvektiven Transport der Zielsubstanz durch die Membran zuläßt.

Bei gegebenem Druckgradienten im Kanalsystem um ein Behandlungselement und gegebenen geometrischen Dimensionen des Behandlungselements wird natürlich der Sekundärstrom maximiert, wenn die mittlere Porengröße der Membran maximiert wird. Im Anwendungsfall muß die Porengröße aber auch auf die Größe der Zielsubstanz abgestimmt werden, die in Form gelöster Makromoleküle, aber auch in Form kleiner Teilchen mit einer Teilchengröße im Sub-Mikrometerbereich vorliegen kann. Gleichzeitig kann es z.B. bei der stoffspezifischen Behandlung von Suspensionen vielfach erforderlich sein, daß die Membran eine trennenden Funktion übernimmt und in der Suspension enthaltende Komponenten, die nicht Zielsubstanzen sind, zurückhält. Dies bedeutet, daß die Porengröße einen bestimmten maximalen Wert nicht übersteigen darf. Hierdurch kann einer Blockade des Porensystems oder beispielsweise unerwünschten Wechselwirkungen zwischen derartigen zurückzuhaltenden Komponenten mit zur Wechselwirkung mit den Zielsubstanzen vorgesehenen stoffspezifisch wirkenden Gruppen in der Membran vorgebeugt werden.

Auf der anderen Seite kann es im Hinblick auf die im folgenden ausgeführten Anwendungen der erfindungsgemäßen Vorrichtung bzw. Ausführungsformen des erfindungsgemäßen Verfahrens eher wichtig sein, Membranen mit möglichst kleinen Porengrößen und möglichst großem Porenvolumen bzw. möglichst großer Porosität zu verwenden, um so für die stoffspezifische Behandlung eine möglichst große innere Oberfläche der Membran zur Verfügung zu stellen. Bevorzugt weisen die erfindungsgemäß eingesetzten Membranen eine mittlere Porosität zwischen 50 Vol.% und 90 Vol.% auf. Als mittlere Porosität wird das Verhältnis des Porenvolumens der Membran zum Membranvolumen verstanden, wobei sich das Membranvolumen aus dem Porenvolumen und dem Volumen des die Membranstruktur aufbauenden Materials zusammensetzt.

Die Anforderungen an den Aufbau der Membran, d.h. an ihre Struktur und Porengrößenverteilung über die Membrandicke resultieren aus dem jeweiligen Anwendungsfall der stoffspezifischen Behandlung. Die Membranstruktur kann über die Dicke isotrop sein, d.h. innerhalb der Membranstruktur sind die Porendurchmesser im wesentlichen konstant, sie können nicht isotrop, symmetrisch oder auch asymmetrisch sein, und die Membran kann an einer ihrer Seiten eine Schicht mit wesentlich dichterer Porenstruktur, d.h. eine Haut aufweisen. Im Falle einer asymmetrischen Membran kann die dichtere Schicht der Außenseite des Behandlungselements oder dem Hohlraum zugewandt sein. Beispielsweise kann es bei der stoffspezifischen Behandlung von Suspensionen erforderlich sein, daß die Membran zur Erzielung einer bestimmten Trennwirkung einen kleinen Porendurchmesser an der der Suspension zugewandten Seite aufweist. Um gleichzeitig einen möglichst großen Sekundärstrom durch die Membran bzw. das Behandlungselement zu erhalten, ist jedoch die übrige Membranstruktur zweckmäßigerweise grobporiger, jedoch je nach Anwendung nicht zu grobporig, um eine möglichst große innere Oberfläche zu erzielen.

Bevorzugt werden Membranen mit einem mittleren Porendurchmesser zwischen 0,005 und 5 µm, besonders bevorzugt solche mit einem mittleren Porendurchmesser zwischen 0,1 und 3 µm eingesetzt.

Zur Bestimmung des mittleren Porendurchmessers werden je nach Größe des Porendurchmessers und je nach Membranstruktur unterschiedliche Verfahren angewandt. Für im wesentlichen isotrope Porenstrukturen werden Porendurchmesser indirekt durch ein Filtrationsexperiment bestimmt, indem eine wässrige Dextranlösung mit einer vorgegebenen Größenverteilung von Dextranmolekülen durch die Membran filtriert wird. Aus dem dabei gemessenen relativen Rückhalt als Funktion der nominalen Moleküldurchmesser wird die Porendurchmesserverteilung und daraus der mittlere Porendurchmesser berechnet. Dieses Verfahren wird beispielsweise von K. Sakai, J. Membrane Science 96 (1994), 91-130, oder von Shin-ichi Nakao, J. Membrane Science 96 (1994) 131-165, für Dialyse- bzw. Filtrationsmembranen beschrieben.

Für nicht-isotrope Membranen, die z.B. eine Schicht mit dichterer Porenstruktur aufweisen, werden zur Bestimmung der mittleren Porendurchmesser innerhalb der dichteren Schicht ebenfalls die zitierten Bestimmungsverfahren basierend auf Filtrationsexperimenten herangezogen. Zur Bestimmung der mittleren Porendurchmesser der grobporigeren Bereiche der nicht-isotropen Membranen wird ein bildanalytisches Verfahren nach L. Zeman u.a., J. Membrane Science 71 (1992), 221-231 eingesetzt. Dieses eignet sich für einen Porengrößenbereich zwischen 0,1 µm und 5 µm, naturgemäß sowohl für isotrope als auch für nicht-isotrope Porenstrukturen.

Es ist für Anwendungen der erfindungsgemäßen Vorrichtung bzw. des erfindungsgemäßen Verfahrens für Flüssigkeiten wie insbesondere klare Lösungen oder Suspensionen vorteilhaft, wenn die Membran in Richtung ihrer Erstreckung zwischen Kanalsystem und dem mindestens einen Hohlraum, d.h. senkrecht zu ihrer äußeren Oberfläche in mindestens 80 % dieser Erstreckung einen im wesentlichen konstanten mittleren Porendurchmesser aufweist. Hierdurch läßt sich eine hohe innere Oberfläche, verbunden mit einer großen Anzahl immobilisierter, stoffspezifisch wirkender Gruppen in der Membran bei gleichzeitig-geringem Druckverlust bei der Durchströmung der Membran senkrecht zu ihrer Oberfläche und damit ein hoher Sekundärstrom erzielen. Als ein im wesentlichen konstanter mittlerer Porendurchmesser wird ein solcher verstanden, der sich in der genannten Erstreckung der Membran um nicht mehr als +/-50 % ändert.

Für die stoffspezifische Behandlung von Suspensionen erweist es sich als günstig, wenn eine Membran eingesetzt wird, die auf ihrer dem Kanalsystem zugewandten Seite eine Schicht besitzt, die einen kleineren mittleren Porendurchmesser aufweist als der in Richtung des Hohlraums angrenzende Bereich der Membran mit im wesentlichen konstantem mittleren Porendurchmesser. Vorteilhafterweise ist diese Schicht zwischen 1 µm und 5 µm dick und weist einen mittleren Porendurchmesser auf, der um den Faktor 5 bis 50 kleiner ist als der mittlere Porendurchmesser im angrenzenden Bereich.

In der erfindungsgemäßen Vorrichtung bzw. zur Durchführung des erfindungsgemäßen Verfahrens werden bevorzugt poröse Membranen mit großer innerer Oberfläche eingesetzt. Bewährt haben sich poröse Membranen mit einer BET-Oberfläche zwischen 2 und 300 m² je cm³ Membranvolumen, bestens bewährt haben sich solche Membranen mit einer BET-Oberfläche zwischen 4 und 30 m² je cm³ Membranvolumen. Das auf Stickstoffadsorptionsmessung basierende BET-Verfahren zur Bestimmung der Oberfläche poröser Membranstrukturen ist von K.Kaneko, J. Membrane Science 96 (1994), 59-89 beschrieben.

Im Rahmen der vorliegenden Erfindung werden bevorzugt Hohlfasermembranen oder Flachmembranen eingesetzt, um die Behandlungselemente auszubilden. Es sind aber auch andere Membranformen wie z.B. Membranschläuche oder Membranrohre eingeschlossen.

Im Falle der bevorzugten Verwendung von Hohlfasermembranen ist die Wand der Hohlfasermembran gleichzeitig auch Wand des mindestens einen Hohlraums des Behandlungselements und die Hohlfasermembran mindestens an einem Ende geschlossen. Der Hohlraum wird durch das Lumen der Hohlfasermembran ausgebildet und von der Innenseite der Hohlfasermembran begrenzt.

Aus Hohlfasermembranen lassen sich auf einfache Weise Behandlungselemente im Sinne der vorliegenden Erfindung herstellen. Durch Verschließen beider Enden oder nur eines Endes von Hohlfasermembranstücken, beispielsweise durch Abschweißen, lassen sich Behandlungselemente mit geschlossenem oder mit an einer Seite geöffnetem Hohlraum herstellen. Durch Falten von Hohlfasermembranstücken quer zur Längsachse entstehen je nach Ausführung Behandlungselemente mit zwei einseitig geöffneten Hohlräumen oder Behandlungselemente mit einem Hohlraum, der zwei in die gleiche Richtung weisende Öffnungen besitzt. Derartige Behandlungselemente sind so in das Gehäuse der erfindungsgemäßen Vorrichtung einzubringen, daß die Öffnungen in Richtung der Auslaßeinrichtung der Vorrichtung weisen und die Behandlungselemente stets in Richtung der Einlaßeinrichtung verschlossen sind.

Es können Hohlfasermembranen mit verschiedenen äußeren Konturen, d.h. mit im Querschnitt betrachtet verschiedenen äußeren Umrissen eingesetzt werden. Die Hohlfasermembranen können beispielsweise eine im wesentlichen runde bzw. kreisförmige, dreieckige, viereckige, sechseckige oder achteckige Kontur aufweisen, sie können auch oval, eliptisch, dreilappig, vierlappig usw. ausgebildet sein. Für den Einsatz in der erfindungsgemäßen Vorrichtung bzw. zur Durchführung des erfindungsgemäßen Verfahrens haben sich Hohlfasermembranen bewährt, die eine Wandstärke zwischen 15 µm und 500 µm aufweisen, bestens bewährt haben sich Hohlfasermembranen mit einer Wandstärke zwischen 100 µm und 300 µm. Vorzugsweise beträgt der hydraulische Durchmesser der eingesetzten Hohlfasermembranen 50 µm bis 900 µm, besonders bevorzugt sind Hohlfasermembranen mit einem hydraulischen Durchmesser zwischen 200 µm und 400 µm.

Die Hohlfasermembranen sind vorteilhafterweise zu mindestens einer Gruppe von nebeneinanderliegenden Behandlungselementen zusammengefaßt, wobei die Hohlfasermembranen einer solchen Gruppe durch textile Fäden auf Abstand gehalten werden. Im Prinzip genügt es dabei, die textilen Fäden zwischen die Hohlfasermembranen zu legen. Bevorzugt werden jedoch die Hohlfasermembranen einer Gruppe mittels der textilen Fäden in mindestens einer Hohlfasermatte eingebunden. Derartige Matten lassen sich vorteilhaft nach bekannten Verfahren als Wirkmatte, Webmatte oder Webbändchen, aber auch als Strick- oder als Häkelmatte herstellen. In den Fällen des Webens oder Wirkens sind die textilen Fäden die quer zu den Hohlfasermembranen verlaufenden Web- bzw. Wirkfäden. Durch diese Querfäden werden die Hohlfasermembranen vorteilhaft gegeneinander auf gleichmäßigen Abstand gehalten und innerhalb der Matten im wesentlichen parallel zueinander angeordnet. Mittels derartiger Matten lassen sich Gruppen von nebeneinanderliegenden Behandlungselementen herstellen, die eine hohe Ordnung aufweisen und bei denen zwischen den Behandlungselementen ein gleichmäßiges Kanalsystem ausgebildet ist.

Derartige Matten von Behandlungselementen aus Hohlfasermembanen können beispielsweise einlagig oder auch mehrlagig nach bekannten Verfahren spiralförmig zu Bündeln aufgewickelt werden oder zu Stapeln von einzelnen Mattenlagen oder gefalteten Mattenlagen aufeinandergeschichtet werden. Dabei werden die einzelnen Wicklungen bzw. die einzelnen Lagen zweckmäßigerweise gegeneinander auf Abstand gehalten, z.B. mittels der eingewebten oder eingewirkten Fäden und gegebenenfalls mittels zusätzlich zwischen den einzelnen Wikkellagen eingebrachter Abstandshalter etwa in Form von fluiddurchlässigen Vliesen oder Geweben.

Die so hergestellten Gruppen von Hohlfasermembranen werden anschließend in das Gehäuse der erfindungsgemäßen Vorrichtung eingebracht. Hierbei ist das Vorhandensein einer elastischen Komponente der Abstandshalter, wie sie beispielsweise bei Vliesen aufgrund einer reversiblen Kompressibilität der Vliese auftritt, von Vorteil und erleichtert das Einbringen der Gruppen in das Gehäuse. Vorzugsweise werden Bündel verwendet, bei denen mindestens eine Hohlfasermatte um eine Achse oder einen Kern parallel zur Richtung der Erstreckung des Kanalsystems zwischen Einlaßeinrichtung und Auslaßeinrichtung der Vorrichtung spiralförmig aufgewickelt ist. Eine weitere vorteilhafte Ausführungsform sind Bündel, bei denen mindestens zwei Hohlfasermatten übereinandergelegt und um eine Achse oder einen Kern parallel zur Richtung der genannten Erstreckung des Kanalsystems spiralförmig aufgewickelt sind, wobei die Hohlfasermatten so übereinandergelegt sind, daß die Hohlfäden der übereinandergelegten Hohlfasermatten in eine sich überkreuzende Anordnung gebracht sind. Die Herstellung derartiger Bündel wird eingehend in der EP 285 812 beschrieben. Für solche Bündel kann der sich zwischen den überkreuzt angeordneten Hohlfasermembranen ausbildende Winkel zwischen 0° und 120° liegen, als günstig haben sich Winkel zwischen 30° und 90° herausgestellt.

Aus Hohlfasermembranen können auch auf einfache Weise Behandlungselemente mit mehr als einem Hohlraum hergestellt werden. Entsprechend in der Länge bemessene Hohlfasermembranstücke können in ihrer Längsachse beispielsweise an mehreren Stellen zusammengepreßt oder verschweißt werden, wodurch mehrere hintereinanderliegende geschlossene Hohlräume abgeteilt werden. In ähnlicher Weise lassen sich auch mehrere geöffnete Hohlräume realisieren, indem die Hohlfasermembran an mehreren Stellen entlang ihrer Längsachse quer zu dieser teilweise eingeschnitten wird, wobei - bezogen auf den in der Vorrichtung eingebauten Zustand eines so hergestellten Behandlungselements - die in Richtung der Auslaßeinrichtung liegende Schnittkante mit der stehengebliebenen Membranwand verschweißt ist, um so die Hohlräume in Richtung der Einlaßeinrichtung zu schließen.

Gemäß einer weiteren bevorzugten Ausführungsform der Erfindung ist das mindestens eine Behandlungselement aus mindestens einer Flachmembran gebildet. Ein solches Behandlungselement läßt sich beispielsweise durch U-förmiges Falten eines beispielsweise rechteckigen oder quadratischen Stücks einer Flachmembran herstellen. Die beiden entstehenden Schenkel der U-förmig gefalteten Flachmembran werden zur Bildung des mindestens einen Hohlraums auf Abstand gehalten. Als Abstandshalter kann ein für Fluide durchlässiges Material wie z.B. ein Vlies oder ein Gewebe eingesetzt werden.

Gemäß einer weiteren vorteilhaften Ausbildung eines Behandlungselements aus Flachmembranen werden zwei zueinander parallele und gegeneinander auf Abstand angeordnete Flachmembranen mindestens an ihrer in Richtung zur Einlaßeinrichtung weisenden Kante formschlüssig miteinander verbunden, beispielsweise miteinander verschweißt, so daß durch die Flachmembranen die Schenkel des Behandlungselements ausgebildet werden. Die beiden eingesetzten Flachmembranen können gleich sein, sie können jedoch auch unterschiedlich sein wie z.B. im Hinblick auf ihr Material, ihre Struktur oder die zur Wechselwirkung mit den Zielsubstanzen vorgesehenen stoffspezifisch wirkenden Gruppen. Auch kann eine der beiden Flachmembranen gegen eine beispielsweise fluidundurchlässige Folie eingetauscht werden, wo dies - etwa aus Gründen der Stabilität der Behandlungselemente oder aus Fertigungsgesichtspunkten - günstig erscheint.

Bei solchen Behandlungselementen wird die den Hohlraum umschließende Seite der Membran bzw. gegebenenfalls eingesetzten Folie als Innenseite und die nach außen weisende Seite als Außenseite der Membran bzw. der gegebenenfalls eingesetzten Folie definiert.

Die in der erfindungsgemäßen Vorrichtung bzw. zur Durchführung des erfindungsgemäßen Verfahrens eingesetzten Flachmembranen weisen vorzugsweise eine Wandstärke zwischen 15 µm und 500 µm auf, besonders bevorzugt sind Flachmembranen mit einer Wandstärke zwischen 100 µm und 300 µm.

Die beiden an die Faltkante bzw. die formschlüssig verschlossene Kante angrenzenden Seiten des Behandlungselements sind zweckmäßigerweise z.B. durch Verschweißen ebenfalls verschlossen. Seitlich offene Behandlungselemente aus Flachmembranen sind wegen möglicher Leck- und Kurzschlußströme bei der Anwendung nicht empfehlenswert. Die der Faltkante gegenüberliegende Kante kann zur Herstellung eines geschlossenen Hohlraums ebenfalls verschlossen werden. Sie bleibt aber vorteilhafterweise zur Herstellung eines an einer Seite geöffneten Hohlraums unverschlossen, wobei in diesem Fall das so entstandene Behandlungselement so in das Gehäuse der erfindungsgemäßen Vorrichtung einzubringen ist, daß die Öffnung in Richtung der Auslaßeinrichtung des Gehäuses weist.

Solchermaßen hergestellte Behandlungselemente können in ebener Form eingesetzt werden, wobei bevorzugt mehrere dieser ebenen Behandlungselemente zu Gruppen stapelförmig nebeneinandergeschichtet sind. Zweckmäßigerweise wird zur Abstandshaltung zwischen den Behandlungselementen einer Gruppe und zwischen der Gehäuseinnenwand und den daran angrenzenden Behandlungselementen ein für Fluide durchlässiges Material wie z.B. ein Gewebe oder ein Vlies eingesetzt, an das insbesondere bei der stoffspezifischen Behandlung von Suspensionen hohe Anforderungen hinsichtlich Durchlässigkeit zu stellen sind. Dieses durchlässige Material hält also die Außenseiten der Membranen und der gegebenenfalls eingesetzten Folien auf Abstand und baut darüberhinaus in Strömungsrichtung des Primärstroms einen zusätzlichen Druckgradienten auf, der Einfluß auf die Größe des Sekundärstroms hat.

Die Abstandshalter, die, wie beschrieben, für einen definierten Abstand zwischen den Schenkeln der zumindest teilweise aus Flachmembranen ausgebildeten Behandlungselemente, zwischen den Behandlungselementen einer Gruppe sowie zwischen Gehäuseinnenwand und daran angrenzenden Behandlungselementen sorgen sollen und gleichzeitig fluiddurchlässig sein sollen, können als separate Elemente ausgebildet sein. Die Abstandshalterfunktion auf der Innenseite und/oder der Außenseite der Membran und/oder der gegebenenfalls mit der Membran verbundenen Folie kann jedoch auch in die Membran bzw. die Folie selbst integriert werden z.B. durch rillenförmige, noppenförmige oder andere profilierte Oberflächenstrukturen.

Gruppen von Behandlungselementen aus Flachmembranen lassen sich beispielsweise durch Plissieren eines dreilagigen flächigen Laminats herstellen, das aus einem flächigen Abstandshalter, etwa in Gestalt eines durchlässigen Vlieses, einer mittig angeordneten Flachmembranlage und einer weiteren Abstandshalterlage besteht. Durch gemeinsames Plissieren dieses dreilagigen Laminats ergibt sich ein ebenes Plissierprodukt, aus dem durch anschließendes, in Bezug auf die Erstreckungsebene des Plissierprodukts z.B. mittiges Aufschneiden Gruppen von nebeneinander angeordneten Behandlungselementen aus U-förmig gefalteten Flachmembranen entstehen. Zur besseren Verarbeitung können die drei Lagen des Laminats z.B. auch punktförmig miteinander verbunden werden.

Bewährt hat sich eine Ausführungsform, bei der die Faltkanten der Behandlungselemente zweier aufeinanderfolgender Stufen der erfindungsgemäßen Vorrichtung in Richtung der Erstreckung des Kanalsystems zwischen Einlaßeinrichtung und Auslaßeinrichtung gesehen einen Winkel zwischen 5° und 175° ausbilden. Bevorzugt sind die Winkel 30°, 45 ° oder 90°. Auf diese Weise wird eine bessere Durchmischung zwischen Primärstrom und Sekundärstrom nach jeder Stufe erreicht und die Ausbildung einer Randströmung zwischen Behandlungselementen und Gehäuseinnenwand reduziert. Der Abstand zwischen den Stufen kann minimiert werden und liegt bevorzugt so, daß das Verhältnis s/D des Abstands s zwischen zwei benachbarten Stufen zum hydraulischen Durchmesser D des Hohlraums der Behandlungselemente zwischen 0 und 1 liegt.

Aus Flachmembranen lassen sich auch auf einfache Weise Behandlungselemente mit mehreren hintereinanderliegenden Hohlräumen herstellen. Entsprechend in ihrer Länge in Durchströmrichtung des Gehäuses der erfindungsgemäßen Vorrichtung bemessene Flachmembran-Behandlungselemente können in ihrer Längserstreckung beispielsweise an mehreren Stellen quer zur Längserstreckung zusammengepreßt oder zusammengeschweißt werden, wodurch mehrere hintereinanderliegende, geschlossene und durch Abstandshalter abgestützte Hohlräume entstehen. In ähnlicher Weise können auch mehrere hintereinanderliegende, in Richtung der Auslaßeinrichtung geöffnete Hohlräume realisiert werden, indem die Behandlungselemente an mehreren Stellen entlang ihrer Längsachse quer zu dieser an ihrem einen Schenkel durchgeschnitten werden, und die in Richtung der Auslaßeinrichtung liegende Schnittkante mit dem stehengebliebenen Schenkel des Behandlungselements verschweißt oder verklebt wird, um so die Hohlräume in Richtung der Einlaßeinrichtung zu schließen.

Die beschriebenen flachen Behandlungselemente lassen sich auch zu spiralförmig gewickelten Behandlungselementen weiterverarbeiten, wobei beispielsweise um eine Achse oder einen Kern senkrecht zur Faltkante bzw. formschlüssig verbundenen Kante spiralförmig gewickelt wird. Zwischen den einzelnen Wickellagen werden vorteilhafterweise Abstandshalter eingebracht, wobei für den Abstand zwischen den einzelnen Wickellagen die gleichen Bedingungen gelten wie für den Abstand zwischen zwei ebenen Behandlungselementen aus Flachmembranen. Solche spiralförmig gewickelten Behandlungselemente werden dann vorteilhafterweise so in einem Gehäuse angeordnet, daß die Richtung der Wickelachse und die Durchströmrichtung des Gehäuses übereinstimmen. Die gegebenenfalls vorhandene geöffnete Kante des spiralförmigen Behandlungselements weist dabei in Richtung der Auslaßeinrichtung des Gehäuses.

Die Form des inneren Querschnitts des Gehäuses, in dem die Behandlungselemente, die zu Gruppen zusammengefaßten Behandlungselemente oder die Stufen von Behandlungeslementen angeordnet sind, kann beliebig sein. Bevorzugt werden jedoch bei Hohlfasermembranen und Flachmembranen Gehäuse mit quadratischem, rechteckigem, sechseckigem, achteckigem oder auch rundem Innenquerschnitt verwendet.

In diese Gehäuse werden die Behandlungselemente bzw. Gruppen von Behandlungselementen, die bevorzugt Abstandshalter mit einer elastischen Komponente aufweisen, unter einer geringen Kompression eingebracht und unter Entspannung im Gehäuse positioniert. Im Falle von ebenen Behandlungselementen aus Flachmembranen können dabei je nach Form des Gehäuseinnenquerschnitts in einfacher Weise definierte Winkel zwischen den Faltkanten der einzelnen Stufen im Gehäuse eingestellt werden.

Bei Behandlungselementen aus Hohlfasermembranen kann je nach Anwendung auch eine Spritzennadel oder eine Kanüle als Gehäuse geeignet sein, in der z.B. auch mehrere Stufen von einzelnen Behandlungselementen hintereinander angeordnet sind. Für andere Anwendungen hat sich ein biegsames Gehäuse z.B. aus einem elastischen Schlauch bewährt. Das Gehäuse kann zum leichteren Einbringen der Behandlungselemente oder der Gruppen oder Stufen von Behandlungselementen auch radial schrumpfbar ausgebildet sein, wobei die Schrumpfung nach Einbringen der Behandlungselemente vorgenommen wird. Besonders bei langen Gehäusen relativ zu ihrem Durchmesser kann es vorteilhaft sein, das Gehäuse beispielsweise wendelförmig oder spiralförmig zu wickeln bzw. zu formen.

Eine feste Verbindung zwischen den Behandlungselementen und dem Gehäuse erübrigt sich in vielen Fällen. Es ist jedoch in Sonderfällen auch möglich, z.B. mittels Polyurethan, Epoxidharz, Thermoplast oder dergleichen die der Gehäusewand benachbarten Behandlungselemente einer Gruppe oder einer Stufe zumindest teilweise fest mit der Gehäuseinnenwand zu verbinden. Auf diese Weise kann für eine stabile Positionierung der jeweiligen Gruppe von Behandlungselementen gesorgt werden, vor allem aber läßt sich so eine unerwünschte Randströmung zwischen Gehäuseinnenwand und Behandlungselementen zumindest verringern, und Ungenauigkeiten in der Außenkontur von Gruppen von Behandlungselementen können ausgeglichen werden. Ferner können dadurch beispielsweise die beschriebenen Flachmembran-Behandlungselemente an ihren Stirnseiten, d.h. an den an die Faltkante bzw. formschlüssig verbundene Kante angrenzenden Kanten abgedichtet werden. Geeignete Methoden, so die Behandlungselemente mit dem Gehäuse zu verbinden, sind beispielsweise in der EP-A-521 495 beschrieben.

Das erfindungsgemäße Verfahren kann zu verschiedensten stoffspezifischen Behandlungen von Fluiden eingesetzt werden. Hierbei werden dadurch besonders gute Resultate in der Behandlung erzielt, dass bei diesen erfindungsgemäßen Verfahren die erfindungsgemäße Vorrichtung verwendet wird, wobei auf und/oder in der Membran stoffspezifisch wirkende Gruppen immobilisiert sind. Im Hinblick auf das jeweilige Behandlungsverfahren können auch unterschiedliche stoffspezifisch wirkende Gruppen auf und/oder in den Membranen immobilisiert sein, die spezifisch mit verschiedenen in dem zu behandelnden Fluid enthaltenen Zielsubstanzen wechselwirken. In gleicher Weise können auch unterschiedliche Membranen mit jeweils verschiedenen stoffspezifisch wirkenden Gruppen zusammen verwendet werden.

In einer bevorzugten Ausgestaltung des erfindungsgemäßen Verfahrens wird das zu behandelnde Fluid rezirkuliert und durchläuft das Behandlungsverfahren mehrfach, bis ein gewünschter Behandlungsgrad erreicht ist. Als zu behandelnde Fluide gelangen bevorzugt partikelhaltige Suspensionen zum Einsatz.

Gemäß einer besonders bevorzugten Ausgestaltung der Erfindung handelt es sich bei den stoffspezifisch wirkenden Gruppen um Liganden zur affinen Trennung von Ligaten aus zu behandelnden Flüssigkeiten, um Enzyme oder um Katalysatoren. Bevorzugte erfindungsgemäße Verfahren sind Verfahren zur Reinigung/Trennung von Ligaten aus einer ligathaltigen Flüssigkeit, wobei Membranen ausgewählt werden, auf und/oder in denen Liganden für besagte Ligaten immobilisiert sind, desweiteren Verfahren zur enzymatischen Behandlung von Flüssigkeiten, wobei Membranen ausgewählt werden, auf und/oder in denen Enzyme immobilisiert sind, sowie Verfahren zur katalytischen Behandlung von Flüssigkeiten, wobei Membranen ausgewählt werden, auf und/oder in denen Katalysatoren immobilisiert sind.

Zur Immobilisierung stoffspezifisch wirkender Gruppen auf und/oder in den Membranen können die in der Literatur beschriebenen Verfahren eingesetzt werden. Auch hinsichtlich der in Bezug auf die jeweilige stoffspezifische Fluidbehandlung verwendbaren stoffspezifisch wirkenden Gruppen kann auf die in der Literatur beschriebenen zurückgegriffen werden. Verschiedene Möglichkeiten der Immobilisierung der stoffspezifisch wirkenden Gruppen kommen in Betracht, sowohl in Bezug auf den Ort, wo sie immobilisiert sind, als auch in Bezug auf die Art und Weise ihrer Immobilisierung.

So können diese stoffspezifisch wirkenden Gruppen an die Membran adsorptiv oder über kovalente Bindungen gekoppelt sein. Diese Kopplung an die Membran kann sowohl vor Einbau in das Gehäuse als auch nach Einsetzen der Membran als Behandlungselement in das Gehäuse der erfindungsgemäßen Vorrichtung erfolgen. Hierbei können in Abstimmung auf den jeweiligen Anwendungsfall die stoffspezifisch wirkenden Gruppen beispielsweise im wesentlichen homogen an die gesamte Oberfläche der porösen Membran, d.h. sowohl an die äußeren als auch an die inneren, durch die Poren gebildeten Oberflächen gekoppelt, d.h. auf und in der Membran immobilisiert sein. Es kann aber auch erforderlich sein, daß die stoffspezifisch wirkenden Gruppen nur an einen Teil dieser Oberflächen immobilisiert sind, etwa wenn einzelne Bestandteile des zu behandelnden Fluids nicht mit den stoffspezifisch wirkenden Gruppen in Kontakt kommen sollen. In einem solchen Fall ist es zweckmäßig, zum einen durch Auswahl einer Membran mit einer geeigneten Trenngrenze einen Transport dieser Bestandteile durch die Membran zu den immmobilisierten stoffspezifisch wirkenden Gruppen zu vermeiden. Zum anderen ist es dann auch erforderlich, die dem Primärstrom, d.h. dem diese Bestandteile enthaltenden Fluid zugewandte Oberfläche der Membran bzw. des Behandlungselements frei von stoffspezifisch wirkenden Gruppen zu halten. Dies kann etwa durch Passivierung dieser Oberfläche z.B. durch eine Plasmabehandlung vor Ankopplung der stoffspezifisch wirkenden Gruppen erreicht werden.

Es kann jedoch auch ein direkter Einbau von stoffspezifisch wirkenden Gruppen in die Membranmatrix erfolgen, im Falle von Membranen aus polymeren Materialien etwa durch Modifikation des Polymermaterials mit beispielsweise ionischen, hydrophilen oder hydrophoben Gruppen oder durch Einsatz von Polymerblends, bei denen mindestens eine Polymerkomponente stoffspezifisch wirkende Gruppen aufweist.

Eine weitere Möglichkeit besteht darin, derartige stoffspezifisch wirkende Gruppen oder auch solche Gruppen aufweisende Trägersubstanzen oder Partikel in das Porensystem einer Membran beim Herstellungsverfahren der Membran einzulagern oder nachträglich in die fertige Membran z.B. einzuschwemmen. In letzterem Fall weist die Membran zweckmäßigerweise eine asymmetrische Struktur sowie gegebenenfalls eine Haut auf, wobei die Öffnungen der Haut bzw. die Poren der feinporigeren Seite der Membran so bemessen sind, daß die stoffspezifisch wirkenden Gruppen bzw. die genannten Trägersubstanzen oder Partikel nicht hindurchtreten können. Hierbei wird das Einschwemmen sowie die nachfolgende stoffspezifische Fluidbehandlung so durchgeführt, daß die Stoffströme von der offenporigeren Seite der Membran in die Membran eintreten und dadurch die die stoffspezifisch wirkenden Gruppen tragenden Substanzen oder Partikel durch die weniger offenporige Seite zurückgehalten werden. Sinnvollerweise werden derartige Systeme mit eingeschwemmten stoffspezifisch wirkenden Gruppen in Verbindung mit Behandlungselementen eingesetzt, die in Richtung der Auslaßöffnung der die Behandlungselemente enthaltenden Vorrichtung geöffnet sind.

In jedem Fall ist die Porengröße der verwendeten Membran so zu wählen, daß auch trotz der in den Poren immobilisierten stoffspezifisch wirkenden Gruppen die Zielsubstanzen konvektiv von zumindest einem Teil des zu behandelnden Fluids durch die Membran transportiert werden kann.

Hinsichtlich des Materials, aus dem die Membran gemäß der Erfindung aufgebaut ist, sind keinerlei Einschränkungen gegeben. So können Membranen aus anorganischen Materialien wie Glas, Keramik, SiO₂, Kohlenstoff oder Metall, aus organischen Polymeren oder Mischungen daraus eingesetzt werden. Die Polymeren können hydrophilen und/oder hydrophoben Charakter aufweisen, sie können ausgewählt sein aus der Gruppe der cellulosischen Polymeren, wie z.B. Cellulose oder regenerierte Cellulose, modifizierte Cellulose, wie z.B. Celluloseester, Celluloseäther, aminmodifizierte Cellulosen, sowie Mischungen von cellulosischen Polymeren, aus der Gruppe der synthetischen Polymeren wie z.B. Polyacrylnitril und entsprechende Copolymere, Polyurethan enthaltende Polymere, Polyarylsulfone und Polyarylethersulfone, wie z.B. Polysulfon oder Polyethersulfon, Polyvinylidenfluorid, Polytetrafluorethylen, wasserunlösliche Polyvinylalkohole, aliphatische und aromatische Polyamide, Polyimide, Polyetherimide, Polyester, Polycarbonate, Polyolefine, wie Polyethylen, Polypropylen, Polyvinylchlorid, Polyphenylenoxid, Polybenzimidazole und Polybenzimiazolone, sowie daraus gewonnenen Modifikationen, Blends, Mischungen oder Copolymeren dieser Polymeren. Diesen Polymeren bzw. Polymergemischen können weitere Polymere wie z.B. Polyethylenoxid, Polyhydroxyether, Polyethylenglykol, Polyvinylpyrrolidon, Polyvinylalkohol oder Polycaprolacton, oder anorganische Stoffe wie z.B. SiO₂ als Zusatzstoffe beigemischt werden. Im Einzelfall kann die Membran auch z.B. einer Oberflächenmodifikation unterzogen worden sein, um bestimmte Eigenschaften der Membranoberfläche z.B. in Form bestimmter funktioneller Gruppen einzustellen.

Besonders gute Erfahrungen wurden mit Membranen aus lösemittelstabilen und pH-Wert-stabilen Polymeren gemacht, insbesondere mit Membranen aus Polytetrafluorethylen oder Polyvinylidenfluorid sowie daraus gewonnenen Modifikationen, Blends, Mischungen oder Copolymeren. Derartige Membranen werden beispielsweise in der DE-A-39 23 128 beschrieben.

Zur Reinigung/Trennung von Ligaten aus einer ligathaltigen Flüssigkeit mittels Affinitätstrennung oder Affinitätschromatograpie sind zahlreiche Anwendungen bekannt. Unter Affinitätschromatographie werden hier biospezifische Adsorptionen und auch Trennverfahren wie z.B. die Ionenaustauscherchromatographie, die Metallchelatchromatographie, die hydrophobe Chromatographie, Kovalentchromatographie oder auch die direkte Sorption von Molekülen auf ein spezifisches Adsorbermaterial verstanden.

Interessante Anwendungen beziehen sich auf die Reinigung von monoklonalen Flüssigkeiten, auf die Entfernung von Proteasen zur Stabilisierung von biologischen Flüssigkeiten, auf die Gewinnung oder therapeutische Entfernung von Blutplasmabestandteilen aus Blutplasma oder Vollblut, auf die Entfernung von Pyrogenen aus biologischen oder pharmazeutischen Flüssigkeiten, auf die Trennung von Enantiomeren oder auf die Isolierung von Enzymen, um nur einige Beispiele zu nennen.

Weitere Anwendungen finden sich im Bereich der Zellselektion. Hierbei läßt sich Gebrauch machen von dem Merkmal, daß beim Einsatz der erfindungsgemäßen Vorrichtung bzw. bei der Durchführung des erfindungsgemäßen Verfahrens ein Teil des Primärstroms als Sekundärstrom auf die membranförmige Wand der Behandlungselemente zu und durch die Wände der Behandlungselemente hindurchströmt. Dies hat bei Einsatz von Zellsuspensionen den Vorteil, daß Zellen, die Zielsubstanz sind, konvektiv auf die Membranen zu transportiert und dort in direkten Kontakt mit z.B. auf der Außenseite der Membran bzw. des Behandlungselements immobilisierten Liganden gebracht werden, die beispielsweise ein bestimmtes Oberflächenprotein der Zellen erkennen. Auch für Anwendungen im Bereich der Gentechnik sind die erfindungsgemäßen Vorrichtungen bestens geeignet, wenn bei solchen Anwendungen z.B. ein konvektiver Transport von Genen zu beispielsweise auf und/oder in der Membran immobilisierten Viren oder Zellen erreicht werden soll.

Liganden können in dem hier gebrauchten Sinne je nach Anwendung nicht-spezifisch, gruppenspezifisch oder spezifisch wirken (s. E. Klein, "Affinity Membranes", John Wiley & Sons, Inc., 1991). Derartige Liganden sind beispielsweise monoklonale Antikörper, polyklonale Antikörper, Peptide, antigene Substanzen, Glycoproteine, Protein A, Protein G, Enzyme, Rezeptorproteine, Wachstumsfaktoren für Zellen, Hormone, Regulationsproteine, Inhibitoren, Kofaktoren, Heparin, Protamin, Poly-L-Lysine, Biotin, Avitin, Aminosäuren wie Trytophan, Phenylamine, L-Histidine oder Antibiotika. Desweiteren können die Liganden auch Salze wie z.B. Fe₄[Fe(CN)₆]₃ oder Farbstoffe sein. Sie können aber auch hydrophile Gruppen oder ionische Gruppen in der Oberfläche des Membranmaterials selbst sein oder an die Oberfläche gebundene Polymere sein. Beispielhaft, jedoch ohne hierauf einzuschränken, sei auch auf die in der WO 90/04609, WO 90/05018 und EP-A-0 565 978 oder auf die in E. Klein, "Affinity Membranes", John Wiley & Sons, Inc., 1991, genannten Beispiele verwiesen.

Ohne an dieser Stelle die Möglichkeiten erschöpfend aufzuzählen, können die Liganden z.B. durch Oberflächenmodifikation der Membran erzeugt werden, sie können direkt oder über Abstandsmoleküle (Spacer) an die Oberfläche gebunden werden, sie können aber auch über Tentakelsysteme oder Ketten an die Oberfläche gebunden werden, wobei an jede Kette bzw. an jedes Tentakelsystem mehrere Liganden gebunden sein können.

Um die Kapazität insbesondere von Ionenaustauschermembranen zu erhöhen, sind verschiedene an sich bekannte Methoden anwendbar, wobei die Anzahl der ionischen Gruppen, d.h. der Liganden auf der Porenoberfläche der Membranen erhöht wird. Bevorzugt sind die Liganden über Moleküle langkettiger Linearpolymerer an die Membran gekoppelt, wobei die Moleküle der langkettigen Linearpolymeren eine Mehrzahl von Liganden tragen. Die Verwendung langkettiger Linearpolymerer, sogenannter Tentakeln, an deren Armen die Liganden sitzen, beschreibt z.B. W. Müller, J. Chromatogr., Bd. 510 (1990), S. 133. Die Herstellung solcher Tentakel ist beispielsweise bei Tsuneda u.a. (Biotechnol. Prog., Bd. 10 (1994), S. 76-81, und J. Chromatogr. Bd. A 689 (1995), S. 211-218) beschrieben und kann über eine strahleninduzierte Pfropfpolymerisation von einem eine Epoxidgruppe enthaltenden Monomer, wie z.B. Glycidylmethacrylat, mit anschließender chemischer Umsetzung in SO₃H-Gruppen oder Diethylamino-Gruppen erfolgen. Ein anderes Verfahren zur Pfropfung von stickstoffhaltigen polymeren Flachmembranen, das zur Erhöhung der Ionenaustauscherkapazität der erfindungsgemäßen Membran-Behandlungselemente eingesetzt werden kann, wird in der EP-A-0 490 940 beschrieben.

Membranen, die mit polymerisierbaren Doppelbindungen derivatisierte Polyamide gemäß der DE-OS-195 01 726 enthalten, sind für die erfindungsgemäße Vorrichtung bzw. zur Durchführung des erfindungsgemäßen Verfahrens bestens geeignet. Diese derivatisierten Polyamide sind erhältlich durch Umsetzung des Polyamids in einer wässrigen Lösung mit einer Verbindung, die sowohl eine polymerisierbare Doppelbindung als auch einen Oxiranring enthält, und können zu Blockpolymerisaten mit verbesserten Eigenschaften umgesetzt werden.

Für Anwendungen im Bereich der enzymatischen oder allgemein katalytischen Behandlung von Flüssigkeiten können Membranen ausgewählt werden, auf und/oder in denen nach an sich bekannten Methoden Enzyme oder Katalysatoren immobilisiert sind.

Anwendungen im Bereich der enzymatischen Flüssigkeitsbehandlung sind z.B. die enzymatische Veresterung von Ethylglycosid, die enzymatische Hydrolyse von Stärke über Amyloglucosidase, die enzymatische Hydrolyse von Enantiomeren, pflanzlichen Ölen, tierischen Ölen, wie z.B. Fischöl, oder Triglyceriden über Lipasen, der enzymatische Abbau von Proteinen über Proteinasen, der Lactoseabbau in der Milch über Lactase oder der Abbau von Blutbestandteilen über entsprechende Enzyme wie beispielsweise Harnstoff über Urease. Auch Anwendungen, wie sie in der US-A-4 061 141 beschrieben werden, fallen hierunter. Andere Enzyme sowie deren Anwendung und Möglichkeiten der Immobilisierung sind in Ullmanns Encyklopädie der technischen Chemie, 4. Auflage, Bd. 10, S. 475-561, Verlag Chemie, Weinheim 1975, beschrieben. Angaben zu Katalysatoren sowie zu deren Immobilisierung in Membranstrukturen, wie sie auch im Rahmen der vorliegenden Erfindung eingesetzt werden können, sind beispielsweise in der US-A-4 266 026 zu finden.

Im Rahmen der Erfindung können die Hohlräume teilweise oder vollständig mit Funktionsträgern angefüllt sein, ohne daß jedoch die eigentliche Sammel- und Kanalfunktion für den Sekundärstrom verlorengeht. Derartige Funktionsträger können im einfachen Fall, wie bereits beschrieben, in Form eines fluiddurchlässigen Vlieses vorliegen und allein die Funktion eines Abstandshalters haben. Es können aber auch solche Vliese eingesetzt werden, in die zusätzlich Partikel eingebracht sind, die wiederum stoffspezifische Gruppen etwa in Form von Liganden oder Enzymen tragen. Andere Beispiele sind Aktivkohle enthaltende Gewebe oder Vliese oder auch Aktivkohle allein. Es ist auch denkbar, lebende Zellen in die Hohlräume einzubringen, die während der stoffspezifischen Behandlung einer Flüssigkeit geeignete biologisch aktive Moleküle erzeugen oder umsetzen.

In gleicher Weise können auch die sich zwischen den Behandlungselementen befindlichen Abstandshalter außer der Abstandshalterfunktion selbst weitere Funktionen erfüllen. So können - außer auf und/oder in der porösen Membranwand der Behandlungselemente zusätzlich stoffspezifisch wirkende Gruppen an den Abstandshaltern immobilisiert sein. Die Abstandshalter sind zu diesem Zweck vorteilhafterweise aus demselben Polymer oder derselben Polymerfamilie wie das Membranmaterial. Insbesondere für die zwischen Behandlungselementen aus Flachmembranen und zwischen den Matten aus Hohlfasermembranelementen eingebrachten Abstandshalter haben sich hierbei textile Gewebe bewährt, wie sie z.B. von Y. Yang u.a., J. Chromatographie 598 (1992), 169-180, beschrieben werden. Dabei führen Gewebe mit multifilen Fäden mit einem Einzelfadendurchmesser zwischen 1 µm und 30 µm zu besonders guten Ergebnissen. Auch haben sich Gewebe bewährt, die sowohl die Schuß- als auch die Kettfäden in einem Winkel zur Richtung des Primärstroms liegen haben, der einen Wert zwischen 30° und 60° aufweist. Die Abstandshalter auf der Primärstromseite können sich auch von denen in den Hohlräumen unterscheiden und z.B. bei Einsatz von Suspensionen weniger dicht gewebt sein.

Die Erfindung wird nachfolgend anhand der Zeichnungen näher erläutert. Es zeigen in vereinfachter schematischer Darstellungsweise:
- Fig. 1:: Ausführungsform eines Behandlungselements mit geschlossenem Hohlraum aus einer Hohlfasermembran, verschweißte Enden
- Fig. 2:: Ausführungsform eines Behandlungselements mit geschlossenem Hohlraum aus einer Hohlfasermembran, in haarnadelförmige Bögen gelegt
- Fig. 3:: Ausführungsform eines Behandlungselements aus einer Hohlfasermembran mit mehreren hintereinanderliegenden geschlossenen Hohlräumen
- Fig. 4:: Ausführungsform eines Behandlungselements aus einer Hohlfasermembran mit einseitig geöffnetem Hohlraum, dessen eines Ende verklebt ist
- Fig. 5:: Ausführungsform eines Behandlungselements aus einer Hohlfasermembran mit einseitig geöffnetem Hohlraum, dessen eines Ende verschweißt ist
- Fig. 6:: Ausführungsform eines Behandlungselements aus einer Hohlfasermembran mit einseitig geöffnetem Hohlraum, hergestellt durch haarnadelförmiges Biegen eines Hohlfasermembranstücks
- Fig. 7:: Ausführungsform eines Behandlungselements aus einer Hohlfasermembran mit mehreren hintereinanderliegenden, einseitig geöffneten Hohlräumen
- Fig. 8: Gruppe von nebeneinander angeordneten und in einer Matte eingebundenen Behandlungselementen aus Hohlfasermembranen mit einseitig geöffneten Hohlräumen
- Fig. 9: Bündel aus einer spiralförmig gewickelten Matte von Behandlungselementen aus Hohlfasermembranen
- Fig. 10: Geschlossenes Behandlungselement (Segment) aus einer wendelförmig um eine horizontale Achse gelegten Hohlfasermembran, Querschnitt
- Fig. 11:: Geschlossenes Behandlungselement (Segment) aus einer wendelförmig um eine vertikale Achse gelegten Hohlfasermembran mit abgeflachtem Querschnitt
- Fig. 12:: Ebenes, einseitig geöffnetes Behandlungselement aus einer U-förmig gefalteten Flachmembran
- Fig. 13:: Aufeinanderfolgende Stufen aus Gruppen von U-förmig gefalteten Flachmembranen, im Winkel von 90° gegeneinander gedreht
- Fig. 14:: Querschnitt durch eine erfindungsgemäße Vorrichtung

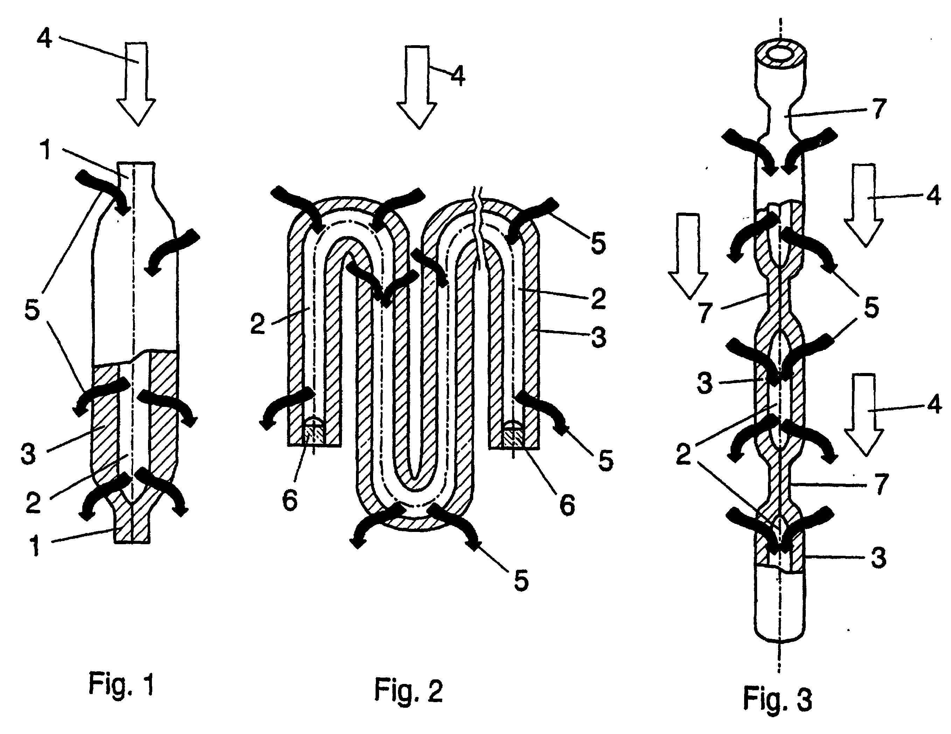

Die Figuren 1 bis 3 zeigen verschiedene Ausführungsformen von Behandlungselementen, die aus Hohlfasermembranen herstellbar sind und geschlossene Hohlräume aufweisen. Hierbei ist in Figur 1 ein teilweise im Schnitt dargestelltes Element gezeigt, das aus einem entsprechend bemessenen Stück einer Hohlfasermembran durch Verschweißen der beiden Enden 1 dieses Stücks hergestellt wurde. Der Hohlraum 2 wird durch das Lumen der Hohlfasermembran gebildet, die Wand 3 des Behandlungselements ist gleichzeitig Wand der Hohlfasermembran. Durch die Pfeile sind der Primärstrom 4 und der Sekundärstrom 5 angedeutet. Der Primärstrom 4 verläuft in Richtung der Längserstreckung des Behandlungselements, der Sekundärstrom 5 tritt im oberen, der Primärstromrichtung entgegengesetzten Teil des Behandlungselements in das Behandlungselement ein, wird im Hohlraum 2 gesammelt und tritt im unteren Teil des Behandlungselements in Richtung des Primärstroms aus dem Behandlungselement aus.

Das Behandlungselement entsprechend Figur 2 kann aus einer Hohlfasermembran erhalten werden, die mehrfach hintereinander in haarnadelförmige Bögen gelegt wurde. In diesem Beispiel sind die Enden des Hohlfaserelements mit einem Verschluß 6 versehen, wie er z.B. durch Eintauchen der Enden in eine Gießmasse oder durch Füllen mit einem Schmelzkleber erhalten werden kann. Natürlich ist auch ein Verschließen der Enden mittels Abschweißen möglich. Der Hohlraum 2 erstreckt sich im dargestellten Fall über die gesamte Länge des Hohlfasermembranstücks. In ähnlicher Weise lassen sich jedoch auch Behandlungselemente durch Knicken der Hohlfasermembran an den Stellen der Bögen herstellen, so daß diese dann an den Knikken abgeschlossene Teilhohlräume aufweisen. Wie für das Behandlungselement in Figur 1 sind auch für dieses Behandlungselement gemäß Figur 2 Primärstrom 4 und Sekundärstrom 5 dargestellt.

Die in Figur 3 dargestellte Ausführungsform ist ein Beispiel für ein Behandlungselement mit mehreren in Richtung des Primärstroms hintereinanderliegenden geschlossenen Hohlräumen 2. Ein solches Element läßt sich beispielsweise durch Abschweißen eines entsprechend bemessenen Hohlfasermembranstücks an mehreren Stellen 7 entlang seiner Längsachse herstellen.

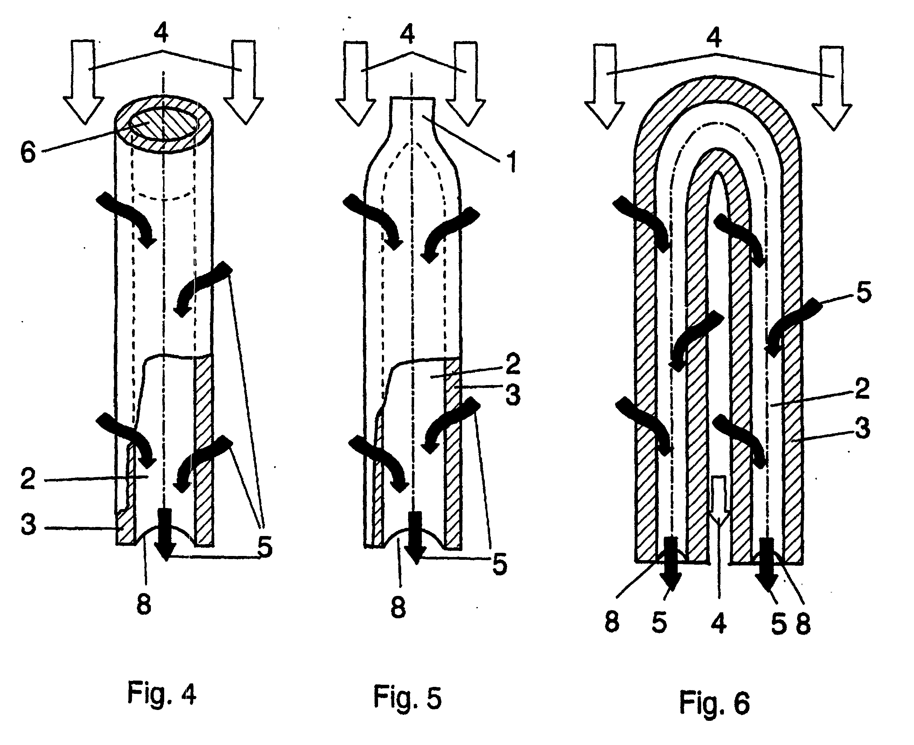

In den Figuren 4 bis 7 sind verschiedene Ausführungsformen von Hohlfasermembran-Behandlungselementen mit einseitig geöffneten Hohlräumen dargestellt, wobei diese Behandlungselemente so in ein erfindungsgemäßes Gehäuse eingebaut werden, daß die Öffnung 8 der einseitig geöffneten Hohlräume in Richtung der Auslaßöffnung, d.h. in Richtung der Durchströmung des Gehäuses durch den Primärstrom 4 weist. Die Ausführungsform gemäß Figur 4 läßt sich aus entsprechend langen Stücken von Hohlfasermembranen durch Verschließen des einen Endes des Hohlfasermembranstücks mit einem Verschluß 6 z.B. mittels eines Schmelzklebers oder durch Eintauchen des Endes in eine Gießmasse herstellen. Die Ausführungsform in Figur 5 stellt ein im wesentlichen gleichartiges Element dar, bei dem jedoch das eine Ende 1 durch Verschweißen verschlossen wurde. Im eingebauten Zustand strömt ein Teil des Primärstroms 4 als Sekundärstrom 5 entlang im wesentlichen der gesamten Länge dieser Behandlungselemente durch die Membranwand 3, sammelt sich in dem in Richtung der Durchströmung des Gehäuses offenen Hohlraum 2 und verläßt am unteren Ende durch die Öffnung 8 das Behandlungselement.

Das in Figur 6 gezeigte Behandlungselement wird durch haarnadelförmiges Biegen eines Hohlfasermembranstücks erhalten und besitzt zwei in der angegebenen Strömungsrichtung des Primärstroms 4 weisende Öffnungen 8 sowie einen durchgehenden teilgeöffneten Hohlraum 2 mit zwei Hälften entsprechend den Hälften des Behandlungselements. Entgegengesetzt zur angegebenen Strömungsrichtung des Primärstroms 4 ist der Hohlraum geschlossen. Anstelle eines haarnadelförmigen Bogens ist auch ein Knick in dem Hohlfasermembranstück vorstellbar, wodurch zwei voneinander getrennte, einseitig geöffnete Hohlräume entstehen.

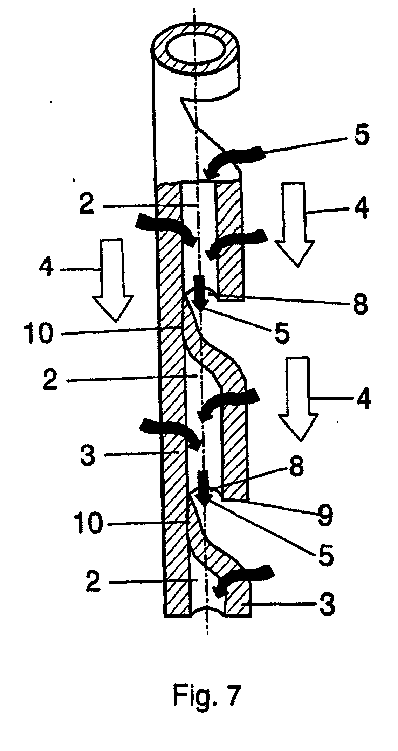

Die in Fig. 7 dargestellte Ausführungsform stellt ein Behandlungselement mit mehreren hintereinander gereihten teilgeöffneten Hohlräumen 2 dar. Diese Hohlräume lassen sich durch teilweises Einschneiden der Hohlfasermembran an mehreren Stellen entlang ihrer Längsachse herstellen, wobei an den Schnittstellen die eine Schnittkante 9 die Öffnung 8 eines Hohlraumes, die andere Schnittkante durch z.B. Verkleben oder Verschweißen mit der stehengebliebenen Wandung der Hohlfasermembran einen Verschluß 10 des angrenzenden Endes des benachbarten Hohlraums ausbildet. Hierbei ist so vorzugehen, das alle Öffnungen 8 eines solchen Behandlungselements in die gleiche Richtung weisen und alle verschlossenen Enden der Hohlräume in die entgegengesetzte Richtung.

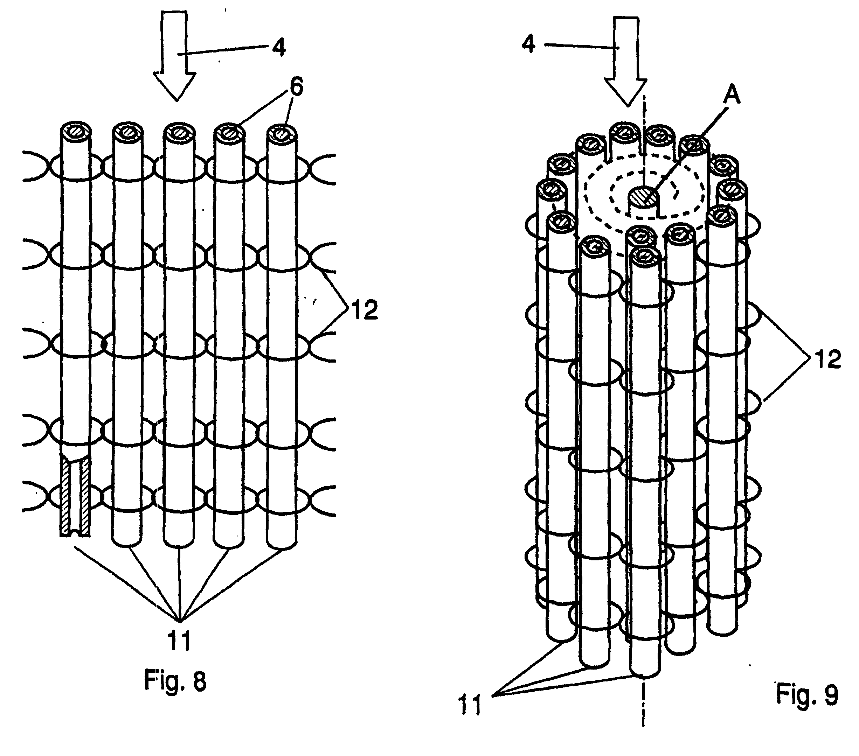

Figur 8 stellt schematisch eine Gruppe von nebeneinander angeordneten Behandlungselementen 11 aus Hohlfasermembranen mit einseitig geöffneten Hohlräumen dar, die mittels textiler Fäden 12 in eine Matte eingebunden sind. Eine solche Matte läßt sich nach bekannten Verfahren beispielsweise mittels Weben oder Wirken herstellen. Die textilen Fäden 12 übernehmen hierbei gleichzeitig die Funktion von Abstandshaltern, und die Hohlfasermembran-Behandlungselemente 11 sind im wesentlichen parallel zueinander angeordnet. Im gezeigten Beispiel ist das der Anströmrichtung des Primärstroms 4 zugewandte Ende der Hohlfasermembranen 11 mit einem Verschluß 6 versehen. Es kann aber auch z.B. durch Verschweißen der Enden oder durch einen Bogen zwischen zwei benachbarten Hohlfasermembranelementen gemäß Fig. 6 verschlossen werden.

Aus einer solchen Matte läßt sich durch spiralförmiges Wikkeln dieser Matte um eine Achse oder einen Kern eine bündelförmige Gruppe von Behandlungselementen herstellen, wie sie schematisch in Figur 9 dargestellt ist. In diesem Beispiel wird die Matte um einen Kern A parallel zu den Hohlfasermembranen spiralförmig aufgewickelt, wobei allein die äußere Wickellage gezeichnet ist. Die Fortsetzung für die weiter innen liegenden Wickellagen ist durch eine spiralförmige, durch die Achsen der im Bündelinneren angeordneten Hohlfasermembranen verlaufende, gestrichelte Linie dargestellt. Die die Hohlfasermembranen 11 verbindenden textilen Fäden 12 halten dabei sowohl die in der Matte nebeneinanderliegenden Hohlfasermembranen als auch die in benachbarten Wickellagen nebeneinanderliegenden Hohlfasermembranen auf Abstand. Bei einem derartigen Aufbau von Gruppen von Behandlungselementen lassen sich eine hohe Ordnung der Behandlungselemente und hohe sowie gleichmäßige Füllgrade der erfindungsgemäßen Vorrichtung realisieren.

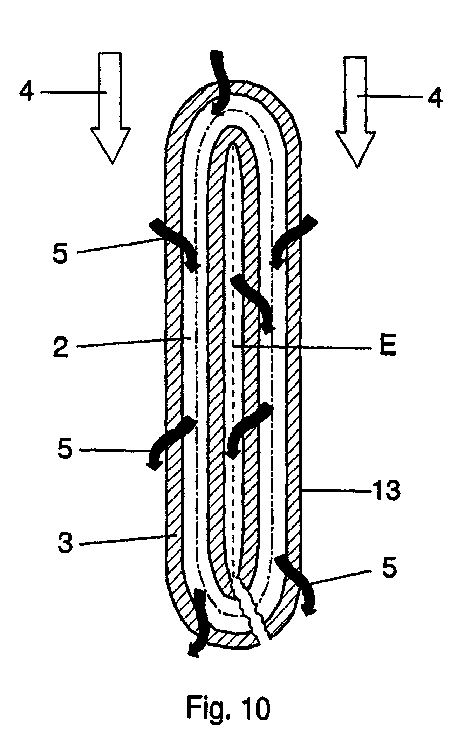

In Fig. 10 ist ein Behandlungselement aus einer Hohlfasermembran dargestellt, welches einen geschlossenen Hohlraum 2 aufweist und sich auf einfache Weise durch wendelförmiges Wickeln einer Hohlfasermembran 13 um eine horizontale Achse bzw., wie dargestellt, um eine Ebene E herstellen läßt, deren eine Erstreckung durch die gestrichelte Linie dargestellt ist, und deren Längserstreckung senkrecht zur Zeichenebene verläuft. Schematisch dargestellt ist der Ausschnitt der Wendel, der in der Zeichenebene liegt. Weitere Windungen schließen sich in der Längserstreckung senkrecht zur Zeichenebene an. Hierbei besteht vorzugsweise zwischen den einzelnen Windungen der Wendel ein Abstand, so daß bei der späteren Anwendung eine gute Umströmung der das Behandlungselement ausbildenden Hohlfasermembran gewährleistet ist. Die Enden der Hohlfasermembran sind verschlossen, so daß insgesamt entlang der Wendel ein sich über die gesamte Länge der Hohlfasermembran erstreckender durchgehender Hohlraum 2 entsteht. In der Anwendung wird dieses wendelförmige Behandlungselement so im Gehäuse angeordnet, daß die Achse der Wendel bzw. die Ebene E, um die diese Wendel gewickelt wurde, in ihrer Längserstreckung quer zur Richtung der Durchströmung des Gehäuses ausgerichtet ist. Hierdurch strömt der Sekundärstrom in dem entgegengesetzt zur Richtung des Primärstroms orientierten Teil der Wendel durch die Wand 3 der Hohlfasermembran in den Hohlraum 2 des Behandlungselements ein und verläßt das Behandlungselement über den in Richtung der Strömung des Primärstroms orientierten Teil.

Ein solches wendelförmiges Behandlungselement läßt sich in einem weiteren Schritt spiralförmig um eine Achse senkrecht zur Achse der Wendel bzw. senkrecht zur Längserstreckung der Ebene, um die die Wendel gewickelt ist, aufwickeln. Dieses spiralförmig gewickelte Behandlungselement kann dann in ein Gehäuse mit zweckmäßigerweise rundem Querschnitt so eingebracht werden, daß die Achse der Spirale in Richtung der Längsachse des Gehäuses weist.

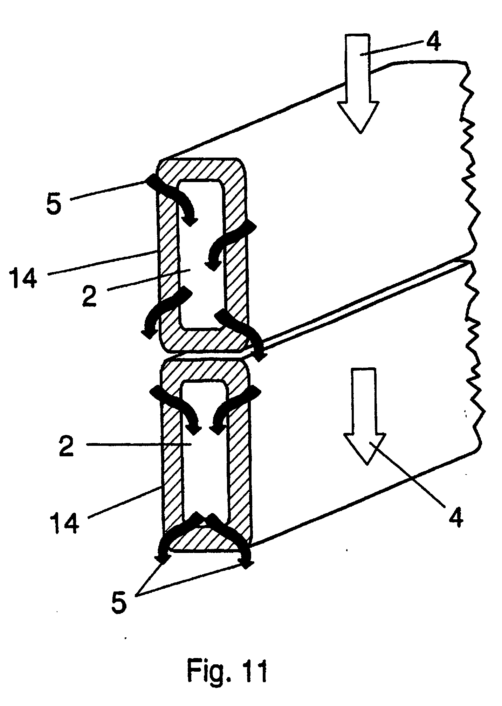

Figur 11 zeigt eine weitere Ausführungsform eines aus einer Hohlfasermembran hergestellten Behandlungselements mit geschlossenem Hohlraum. Im wesentlichen ist der Aufbau dieses Behandlungselements dem des in Figur 10 dargestellten ähnlich, d.h. die Hohlfasermembran ist auch hier wendelförmig um eine Achse bzw. um eine Ebene gewickelt, die parallel zur Richtung des Primärstroms 4 verläuft, wobei die Enden der die Wendel aufbauenden Hohlfasermembran verschlossen sind. Beispielhaft ist dabei bei der Ausführungsform gemäß Figur 11 eine in ihrem Querschnitt abgeflachte Hohlfasermembran dargestellt. In der Figur 11 sind Ausschnitte aus zwei nebeneinanderliegenden Windungen 14 der Wendel dargestellt, wobei die in der Schnittdarstellung sichtbaren Hohlraumquerschnitte aufgrund des wendelförmigen Aufbaus des Elements Querschnitte desselben Hohlraums 2 sind.

Der Einbau dieses Behandlungselements im Gehäuse einer erfindungsgemäßen Vorrichtung erfolgt im Unterschied zu dem Behandlungselement gemäß Figur 10 derart, daß die Achse der Wendel bzw. die Längserstreckung der Ebene, um die die Hohlfasermembran wendelförmig gewickelt ist, in Richtung der Längsachse des Gehäuses und damit in Richtung der Durchströmung des Gehäuses weist. Hierdurch kommt es im wesentlichen zu einer Queranströmung der einzelnen Windungen der Wendel durch den Primärstrom 4. Der Sekundärstrom 5 dringt durch den entgegengesetzt zur Richtung des Primärstroms orientierten Teil der abgeflachten Hohlfasermembran in das Behandlungselement ein, sammelt sich in dem sich über die gesamte Hohlfasermembranlänge erstreckenden Hohlraum und tritt auf der in Strömungsrichtung des Primärstroms 4 orientierten Seite der abgeflachten Hohlfasermembran aus dem Behandlungselement aus. Gleichzeitig entsteht aufgrund der Gesamterstreckung des Behandlungselements entlang seiner Wickelachse im Hohlraum des Behandlungselements ein Fluß in Richtung der Längserstreckung der Hohlfasermembran.

Auch ein derartiges Behandlungselement läßt sich fortbilden, indem z.B. mehrere Lagen aus wendelförmig um eine Achse aufgewickelten Hohlfasermembranen zueinander konzentrisch übereinander angeordnet werden, wobei die einzelnen Lagen z.B. mittels geeigneter Abstandshalter gegeneinander auf Abstand gehalten werden, um eine möglichst gute Umströmung der die einzelnen Lagen aufbauenden Hohlfasermembranen zu erreichen. Eine andere Möglichkeit besteht darin, mindestens eine Hohlfasermembran in mehreren Lagen spiralförmig übereinander um eine Achse oder um einen Kern zu wickeln, wobei innerhalb einer Lage die mindestens eine Hohlfasermembran wendelförmig um die Achse bzw. um den Kern gewickelt ist.

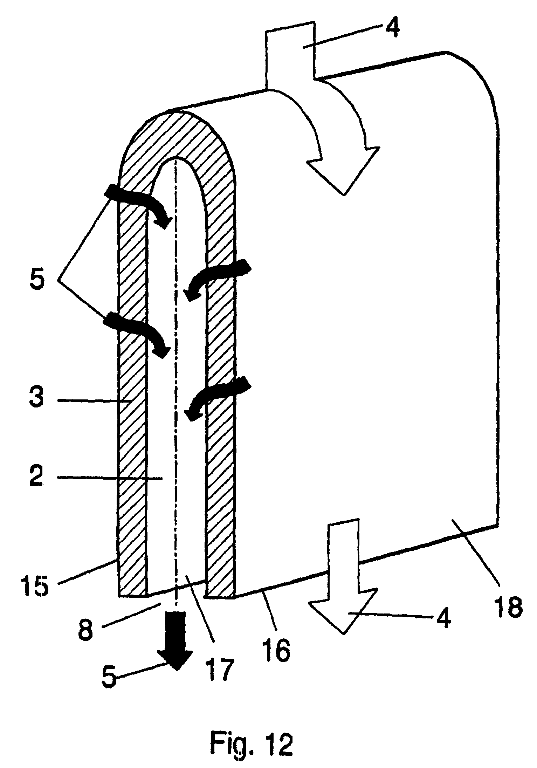

In Figur 12 ist ein Behandlungselement aus einer Flachmembran dargestellt, welches beispielsweise durch U-förmiges Falten eines rechteckigen Stücks einer Flachmembran erhalten wird. Die beiden Schenkel 15 und 16 der gefalteten Flachmembran werden durch einen - hier nicht dargestellten - Abstandshalter z.B. in Form eines flüssigkeitsdurchlässigen Vlieses gegeneinander auf Abstand gehalten. Der Hohlraum 2 dieses Behandlungselements wird durch die Schenkel des Flachmembran-Behandlungselements, die Faltkante sowie die - hier nicht dargestellten ≠ miteinander verbundenen Seitenkanten begrenzt und weist an der der Faltkante gegenüberliegenden Seite eine Öffnung 8 auf. Die dem Hohlraum 2 zugewandte Seite der Flachmembran stellt die Innenseite 17, die andere Seite der Flachmembran die Außenseite 18 dar. Im Anwendungsfall umströmt der Primärstrom 4 ein solches Behandlungselement von seiner Faltkante her an seiner Außenseite. Der Sekundärstrom 5 tritt von der Außenseite her im wesentlichen über ihre gesamte Erstreckung in die Membranwand 3 ein, sammelt sich im Hohlraum 2 des Behandlungselements und tritt an der geöffneten Seite 8 des Hohlraums in Richtung der Durchströmung des dieses Behandlungselement enthaltenen Gehäuses aus dem Behandlungselement als behandelter Sekundärstrom aus.

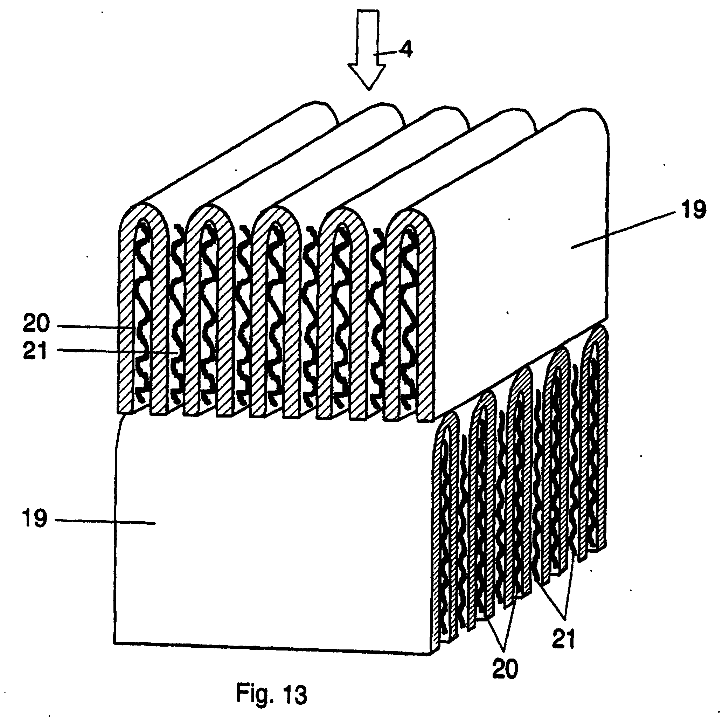

Figur 13 zeigt zwei aufeinanderfolgende Stufen 19 von Gruppen aus stapelförmig nebeneinander angeordneten, U-förmig gefalteten, ebenen Flachmembran-Behandlungselementen. In diesem Beispiel sind die Faltkanten der Behandlungselemente der benachbarten Stufen gegeneinander um 90° gedreht. Dadurch kann auf einen Abstandshalter zwischen den Stufen verzichtet werden, wobei dennoch eine gute Durchmischung von Primärstrom und Sekundärstrom gewährleistet ist. Die Behandlungselemente weisen zwischen den sie ausbildenden Schenkeln für Fluide durchlässige Abstandshalter 20 auf; die benachbarten Behandlungselemente innerhalb einer Stufe werden ebenfalls durch für Fluide durchlässige Abstandshalter 21 auf Abstand gehalten, um so eine freie Umströmbarkeit der Behandlungselemente an ihrer Außenseite durch den Primärstrom 4 zu erreichen.

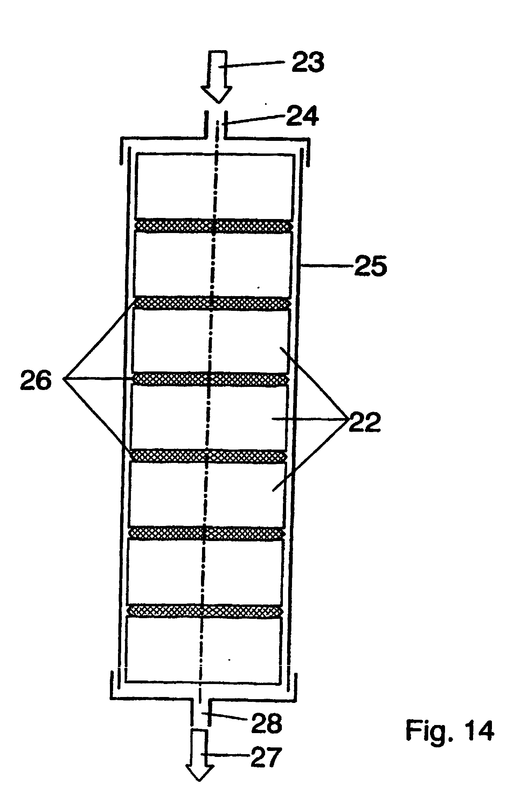

In Figur 14 ist eine mehrere Stufen 22 von Behandlungselementen enthaltende erfindungsgemäße Vorrichtung schematisch im Querschnitt dargestellt. Das zu behandelnde Fluid tritt als Feedstrom 23 durch die Einlaßeinrichtung 24 in das Gehäuse 25 der erfindungsgemäßen Vorrichtung ein und wird, gegebenenfalls unterstützt durch eine entsprechend ausgelegte - hier nicht dargestellte - Verteilereinrichtung, auf die Behandlungselemente der ersten Stufe gleichmäßig verteilt. Das Fluid durchläuft dann nacheinander die im Gehäuse enthaltenen Stufen 22 von Behandlungselementen, in denen die stoffspezifische Behandlung des Fluids erfolgt. Benachbarte Stufen werden in diesem Beispiel durch für das zu behandelnde Fluid durchlässige Abstandshalter 26 gegeneinander auf Abstand gehalten, um vor Eintritt des zu behandelnden Fluids in die nächste Stufe eine gute Durchmischung von Primärstrom und Sekundärstrom zu erreichen. Nach Durchlaufen der erforderlichen Anzahl von Stufen 22 wird das behandelte Fluid 27 über die Auslaßeinrichtung 28 aus dem Gehäuse abgeleitet.

## Patentansprüche

1. Vorrichtung zur stoffspezifischen Behandlung eines Fluids durch Wechselwirkung mit in dem zu behandelnden Fluid enthaltenen Zielsubstanzen, bestehend aus
a) einem Gehäuse (25),
b) einer Einlaßeinrichtung (24) zum Einleiten des zu behandelnden Fluids (23) in das Gehäuse (25),
c) einer Auslaßeinrichtung (28) zum Ableiten des behandelten Fluids (27) aus dem Gehäuse (25),
d) mindestens einem Behandlungselement zur stoffspezifischen Behandlung des Fluids mit einem der Einlaßeinrichtung und einem der Auslaßeinrichtung zugewandten Ende und einer Wand (3), die zumindest teilweise aus mindestens einer semipermeablen Membran mit einer porösen Struktur gebildet ist,
wobei das mindestens eine Behandlungselement mindestens einen durch die Wand (3) des Behandlungselements gebildeten Hohlraum (2) aufweist, wobei der Hohlraum geschlossen oder höchstens in Richtung der Auslaßeinrichtung (28) einseitig geöffnet ist, wobei das mindestens eine Behandlungselement derart im Gehäuse (25) angeordnet ist, daß zwischen Einlaßeinrichtung (24) und Auslaßeinrichtung (28) ein durchgehendes, vom zu behandelnden Fluid durchströmbares, das mindestens eine Behandlungselement berührendes und umgebendes und das mindestens eine Behandlungselement zumindestens an seinem der Einlaßeinrichtung und an seinem der Auslaßeinrichtung zugewandten Ende im wesentlichen umschließendes Kanalsystem ausgebildet ist und wobei auf und/oder in der Membran stoffspezifisch wirkende Gruppen immobilisiert sind.

2. Vorrichtung nach Anspruch 1, wobei mindestens ein Hohlraum (2) in Richtung der Auslaßeinrichtung (28) eine Öffnung (8) aufweist, die in das Kanalsystem mündet.

3. Vorrichtung nach einem oder mehreren der Ansprüche 1 oder 2, **dadurch gekennzeichnet, daß** mindestens ein Hohlraum (2) ein Verhältnis L/D seiner Abmessung L in Richtung der Erstreckung des Kanalsystems zwischen Einlaßeinrichtung (24) und Auslaßeinrichtung (28) und des hydraulischen Durchmessers D seines Querschnitts senkrecht dazu zwischen 2 und 4000 aufweist.

4. Vorrichtung nach einem oder mehreren der Ansprüche 1 bis 3, **dadurch gekennzeichnet, daß** das mindestens eine Behandlungselement ein Verhältnis V_{w}/V_{b} des Volumens seiner Wand (3) V_{w} zu dem aus dem Volumen der Wand (3) V_{w} und dem Volumen des mindestens einen Hohlraums (2) Vₕ zusammengesetzten Volumen des Behandlungselements V_{b} zwischen 0,5 und 0,98 aufweist.

5. Vorrichtung nach einem oder mehreren der Ansprüche 1 bis 4, **dadurch gekennzeichnet, daß** die Wände (3) eines jeden Behandlungselements eine im wesentlichen gleichmäßige Dicke aufweisen.

6. Vorrichtung nach einem oder mehreren der Ansprüche 1 bis 5, **gekennzeichnet durch** mindestens eine Gruppe von mehreren Behandlungselementen, welche Behandlungselemente im wesentlichen quer zur Richtung der Erstreckung des Kanalsystems zwischen Einlaßeinrichtung (24) und Auslaßeinrichtung (28) nebeneinander angeordnet sind.

7. Vorrichtung nach Anspruch 6, **dadurch gekennzeichnet, daß** die in mindestens einer Gruppe zusammengefaßten Behandlungselemente zueinander durch Abstandshalter auf Abstand gehalten werden.

8. Vorrichtung nach Anspruch 7, **dadurch gekennzeichnet, daß** die im Gehäuse (25) angeordneten und der Innenwand des Gehäuses (25) benachbarten Behandlungselemente gegenüber der Gehäuseinnenwand einen Abstand aufweisen, der kleiner oder gleich dem Abstand zwischen den Behandlungselementen ist.

9. Vorrichtung nach einem oder mehreren der Ansprüche 1 bis 8, **dadurch gekennzeichnet, daß** im Gehäuse (25) in Richtung der Erstreckung des Kanalsystems zwischen Einlaßeinrichtung (24) und Auslaßeinrichtung (28) mehrere Behandlungselemente oder Gruppen von Behandlungselementen als Stufen (22) hintereinander angeordnet sind.

10. Vorrichtung nach Anspruch 9, **dadurch gekennzeichnet, daß** die Anzahl der Stufen zwischen 1 und 1000 liegt.

11. Vorrichtung nach Anspruch 10, **dadurch gekennzeichnet, daß** die Anzahl der Stufen zwischen 2 und 100 liegt.

12. Vorrichtung nach einem oder mehreren der Ansprüche 1 bis 11, **dadurch gekennzeichnet, daß** das Verhältnis V_{B}/V_{G} des aus der Summe der Volumina V_{b} der einzelnen Behandlungselemente zusammengesetzte Gesamtvolumen V_{B} aller Behandlungselemente und dem Volumen V_{G} des leeren Gehäuses (25) zwischen 0,4 und 0,95 liegt.

13. Vorrichtung nach Anspruch 12, **dadurch gekennzeichnet, daß** das Verhältnis zwischen 0,55 und 0,75 liegt.

14. Vorrichtung nach einem oder mehreren der Ansprüche 1 bis 13, **dadurch gekennzeichnet, daß** die Membran einen mittleren Porendurchmesser zwischen 0,005 µm und 5 µm aufweist.

15. Vorrichtung nach Anspruch 14, **dadurch gekennzeichnet, daß** die Membran einen mittleren Porendurchmesser zwischen 0,1 µm und 3 µm aufweist.

16. Vorrichtung nach einem oder mehreren der Ansprüche 1 bis 15, **dadurch gekennzeichnet, daß** die Membran in Richtung ihrer Erstreckung zwischen Kanalsystem und dem mindestens einem Hohlraum (2) in mindestens 80 % dieser Erstreckung einen im wesentlichen konstanten mittleren Porendurchmesser aufweist.

17. Vorrichtung nach Anspruch 16, **dadurch gekennzeichnet, daß** die Membran auf ihrer dem Kanalsystem zugewandten Seite eine Schicht besitzt, die einen kleineren mittleren Porendurchmesser aufweist als der in Richtung des mindestens einen Hohlraums (2) angrenzende Bereich der Membran.

18. Vorrichtung nach Anspruch 17, **dadurch gekennzeichnet, daß** die Schicht zwischen 1 µm und 5 µm dick ist.

19. Vorrichtung nach einem oder mehreren der Ansprüche 17 oder 18, **dadurch gekennzeichnet, daß** der mittlere Porendurchmesser in der Schicht um den Faktor 5 bis 50 kleiner ist als im angrenzenden Bereich.

20. Vorrichtung nach einem oder mehreren der Ansprüche 1 bis 19, **dadurch gekennzeichnet, daß** die Membran eine mittlere Porosität zwischen 50 Vol% und 90 Vol% aufweist.

21. Vorrichtung nach einem oder mehreren der Ansprüche 1 bis 20, **dadurch gekennzeichnet, daß** die Membran eine BET-Oberfläche zwischen 2 und 300 m² je cm³ Membranvolumen aufweist.

22. Vorrichtung nach Anspruch 21, **dadurch gekennzeichnet, daß** die Membran eine BET-Oberfläche zwischen 4 und 30 m² je cm³ Membranvolumen aufweist.

23. Vorrichtung nach einem oder mehreren der Ansprüche 1 bis 22, **dadurch gekennzeichnet, daß** die Membran eine Hohlfasermembran und die Wand (3) der Hohlfasermembran Wand des mindestens einen Hohlraums (2) ist und die Hohlfasermembran mindestens an einem Ende geschlossen ist.

24. Vorrichtung nach Anspruch 23, **dadurch gekennzeichnet, daß** die Hohlfasermembranen einer Gruppe von nebeneinanderliegenden Behandlungselementen (11) durch textile Fäden (12) auf Abstand gehalten werden.

25. Vorrichtung nach Anspruch 24, **dadurch gekennzeichnet, daß** die Hohl fasermembranen einer Gruppe mittels der textilen Fäden (12) in mindestens eine Hohlfasermatte eingebunden sind.

26. Vorrichtung nach Anspruch 25, **dadurch gekennzeichnet, daß** die mindestens eine Hohlfasermatte eine Webmatte ist.

27. Vorrichtung nach Anspruch 25, **dadurch gekennzeichnet, daß** die mindestens eine Hohlfasermatte eine Wirkmatte ist.

28. Vorrichtung nach Anspruch 25, **dadurch gekennzeichnet, daß** die mindestens eine Hohlfasermatte ein Webbändchen ist.

29. Vorrichtung nach einem oder mehreren der Ansprüche 25 bis 28, **dadurch gekennzeichnet, daß** die mindestens eine Hohlfasermatte um eine Achse parallel zur Richtung der Erstreckung des Kanalsystems zwischen Einlaßeinrichtung (24) und Auslaßeinrichtung (28) spiralförmig aufgewickelt ist.

30. Vorrichtung nach einem oder mehreren der Ansprüche 25 bis 28, **dadurch gekennzeichnet, daß** mindestens zwei Hohlfasermatten übereinandergelegt und um eine Achse parallel zur Richtung der Erstreckung des Kanalsystems zwischen Einlaßeinrichtung (24) und Auslaßeinrichtung (28) spiralförmig aufgewickelt sind, und die Hohlfasermatten so übereinandergelegt sind, daß die Hohlfäden der übereinandergelegten Hohlfasermatten in eine sich überkreuzende Anordnung gebracht sind.

31. Vorrichtung nach einem oder mehreren der Ansprüche 1 bis 22, **dadurch gekennzeichnet, daß** das mindestens eine Behandlungselement aus mindestens einer Flachmembran gebildet ist.

32. Vorrichtung nach Anspruch 31, **dadurch gekennzeichnet, daß** das mindestens eine Behandlungselement aus einer ü-förmig gefalteten Flachmembran gebildet ist, wobei die durch die Faltung entstandene Faltkante in Richtung der Einlaßeinrichtung (24) angeordnet ist und wobei die so entstandenen Schenkel (15) (16) der U-förmig gefalteten Flachmembran zur Bildung des mindestens einen Hohlraums (2) auf Abstand gehalten werden.

33. Vorrichtung nach Anspruch 31, **dadurch gekennzeichnet, daß** das mindestens eine Behandlungselement aus zwei zueinander parallelen und gegeneinander auf Abstand angeordneten Flachmembranen ausgebildet ist, wobei die beiden Flachmembranen mindestens an ihrer in Richtung zur Einlaßeinrichtung (24) weisenden Kante formschlüssig miteinander verbunden sind.

34. Vorrichtung nach einem oder mehreren der Ansprüche 31 bis 33, **dadurch gekennzeichnet, daß** die mindestens eine Flachmembran spiralförmig um eine Achse parallel zur Richtung der Erstreckung zwischen Einlaßeinrichtung (24) und Auslaßeinrichtung (28) so gewickelt ist, daß die geschlossene Kante in Richtung der Einlaßeinrichtung (24) weist, wobei die Wickellagen durch Abstandshalter auf Abstand gehalten sind und dadurch zwischen den Wickellagen Kanäle ausgebildet sind, die Teil des Kanalsystems sind.

35. Vorrichtung nach einem oder mehreren der Ansprüche 1 bis 34 **dadurch gekennzeichnet, daß** die Membran aus Polytetrafluorethylen oder Polyvinylidenfluorid oder daraus gewonnenen Modifikationen, Blends, Mischungen oder Copolymeren besteht.

36. Vorrichtung nach einem oder mehreren der Ansprüche 1 bis 35, **dadurch gekennzeichnet, daß** die stoffspezifisch wirkenden Gruppen Liganden zur affinen Trennung von Ligaten aus der zu behandelnden Flüssigkeit sind.

37. Vorrichtung nach Anspruch 36, dadurch gekenneichnet, daß die Liganden über Moleküle langkettiger Linearpolymerer an die Membran gekoppelt sind, wobei die Moleküle der langkettigen Linearpolymeren eine Mehrzahl von Liganden tragen.

38. Vorrichtung nach einem oder mehreren der Ansprüche 1 bis 35, **dadurch gekennzeichnet, daß** die stoffspezifisch wirkenden Gruppen Enzyme sind.

39. Vorrichtung nach einem oder mehreren der Ansprüche 1 bis 35, **dadurch gekennzeichnet, daß** die stoffspezifisch wirkenden Gruppen Katalysatoren sind.

40. Verfahren zur stoffspezifischen Behandlung eines Fluids durch Wechselwirkung mit in dem zu behandelnden Fluid enthaltenen Zielsubstanzen unter Verwendung einer Vorrichtung gemäß einem oder mehreren der Ansprüche 1 bis 35, enthaltend mindestens ein Behandlungselement, welches zumindest teilweise aus mindestens einer semipermeablen Membran mit poröser Struktur ausgebildet ist, wobei die Membran mindestens eine erste, die Außenseite definierende Oberfläche sowie mindestens eine zweite, die Innenseite definierende Oberfläche aufweist, umfassend mindestens die Schritte:
a) Einleiten des zu behandelnden Fluids in das Gehäuse,
b) Durchströmen des Gehäuses mit besagtem Fluid, dabei Vorbeileiten des zu behandelnden Fluids als Primärstrom an der Außenseite der Membran, nicht jedoch an ihrer Innenseite derart, daß ein Teil dieses Primärstroms als Sekundärstrom über die Außenseite in die Membran einströmt, durch die Membran hindurchströmt, wobei an dem den Sekundärstrom bildenden Teil des zu behandelnden Fluids die stoffspezifische Behandlung des Fluids erfolgt, und anschließend durch die Innenseite aus der Membran herausströmt,
c) Ausleiten des behandelten Fluids aus dem Gehäuse,
wobei die Membran über ihre gesamte Erstreckung an ihrer Außenseite im wesentlichen frei vom Primärstrom umströmt wird und daß der durch die Membran hindurchgeströmte Sekundärstrom nach der stoffspezifischen Behandlung dem an der Außenseite der Membran strömenden Primärstrom wieder zugeführt wird.

41. Verfahren nach Anspruch 40, **dadurch gekennzeichnet, daß** das zu behandelnde Fluid eine Suspension ist.

42. Verfahren nach einem oder mehreren der Ansprüche 40 oder 41, **dadurch gekennzeichnet, daß** in der Vorrichtung mehrere in Richtung der Durchströmung des Gehäuses räumlich hintereinandergeschaltete Behandlungselemente oder Gruppen von Behandlungselementen als Stufen angeordnet sind und zwischen den einzelnen Stufen der Primärstrom und Sekundärstrom enthaltende Fluidstrom vermischt wird.

43. Verfahren nach einem oder mehreren der Ansprüche 40 bis 42, **dadurch gekennzeichnet, daß** mehrere Gehäuse mit darin angeordneten Behandlungselementen hintereinandergeschaltet werden.

44. Verfahren nach einem oder mehreren der Ansprüche 40 bis 43, **dadurch gekennzeichnet, daß** das zu behandelnde Fluid rezirkuliert wird.

45. Verfahren nach einem oder mehreren der Ansprüche 40 bis 44 zur Reinigung/Trennung von Ligaten aus einer ligathaltigen Flüssigkeit mittels Affinität, **dadurch gekennzeichnet, daß** eine Membran ausgewählt wird, auf und/oder in der Liganden für besagte Ligaten immobilisiert sind.

46. Verfahren nach einem oder mehreren der Ansprüche 40 bis 44 zur enzymatischen Behandlung einer Flüssigkeit, **dadurch gekennzeichnet, daß** eine Membran ausgewählt wird, auf und/oder in der Enzyme immobilisiert sind.

47. Verfahren nach einem oder mehreren der Ansprüche 40 bis 44 zur katalytischen Behandlung von Fluiden, **dadurch gekennzeichnet, daß** eine Membran eingesetzt wird, auf und/oder in der Katalysatoren immobilisiert sind.

## Claims

1. Device for the substance-specific treatment of a fluid by interaction with target substances contained in the fluid to be treated, consisting of
a) a casing (25),
b) an inlet arrangement (24) for introducing the fluid to be treated (23) into the casing (25),
c) an outlet arrangement (28) to remove the treated fluid (27) from the casing (25),
d) at least one treatment element for the substance-specific treatment of the fluid with one end facing the inlet arrangement and the other facing the outlet arrangement and a wall (3) that is at least partially formed from at least one semipermeable membrane with a porous structure,
wherein the at least one treatment element has at least one cavity (2) formed by the wall (3) of the treatment element, wherein the cavity is closed, or open at most at the end facing the outlet arrangement (28), wherein the at least one treatment element is arranged in the casing (25) in such a manner that between the inlet arrangement (24) and the outlet arrangement (28) there is a continuous channel system, through which the fluid to be treated can flow, touching and surrounding the at least one treatment element, and essentially enclosing the at least one treatment element at least at its end facing the inlet arrangement and at its end facing the outlet arrangement, and wherein groups acting in a substance-specific manner are immobilized on and/or in the membrane.

2. Device in accordance with Claim 1, wherein at least one cavity (2) has in the direction of the outlet arrangement (28) an opening (8) which leads into the channel system.

3. Device in accordance with one or more of Claims 1 or 2, **characterized in that** at least one cavity (2) has a ratio L/D of its dimension L in the direction of the extent of the channel system between the inlet arrangement (24) and the outlet arrangement (28) and the hydraulic diameter D of its cross-section perpendicular to it of between 2 and 4000.

4. Device in accordance with one or more of Claims 1 to 3, **characterized in that** the at least one treatment element has a ratio V_{w}/Vₜ of the volume of its wall (3) V_{w} to the volume of the treatment element Vₜ comprising the volume of the wall (3) V_{w} and the volume of the at least one cavity (2) V_{c} between 0.5 and 0.98.

5. Device in accordance with one or more of Claims 1 to 4, **characterized in that** the walls (3) of each treatment element have an essentially uniform thickness.

6. Device in accordance with one or more of Claims 1 to 5, **characterized by** at least one group of several treatment elements, which treatment elements are arranged side by side essentially transverse to the direction of the extent of the channel system between the inlet arrangement (24) and the outlet arrangement (28).

7. Device in accordance with Claim 6, **characterized in that** the treatment elements put together into at least one group are kept apart from one another by spacers.

8. Device in accordance with Claim 7, **characterized in that** the treatment elements arranged in the casing (25) and adjacent to the inner wall of the casing (25) are at a distance from the inner wall of the casing which is less than or equal to the distance between the treatment elements.

9. Device in accordance with one or more of Claims 1 to 8, **characterized in that** several treatment elements or groups of treatment elements are arranged as stages (22) one behind the other in the casing (25) in the direction of the extent of the channel system between the inlet arrangement (24) and the outlet arrangement (28).

10. Device in accordance with Claim 9, **characterized in that** the number of stages is between 1 and 1000.

11. Device in accordance with Claim 10, **characterized in that** the number of stages is between 2 and 100.

12. Device in accordance with one or more of Claims 1 to 11, **characterized in that** the ratio V_{T}/V_{C} of the total volume V_{T} of all treatment elements comprising the sum of the volumes Vₜ of the single treatment elements and the volume V_{C} of the empty casing (25) is between 0.4 and 0.95.

13. Device in accordance with Claim 12, **characterized in that** the ratio is between 0.55 and 0.75.

14. Device in accordance with one or more of Claims 1 to 13, **characterized in that** the membrane has an average pore diameter between 0.005 µm and 5 µm.

15. Device in accordance with Claim 14, **characterized in that** the membrane has an average pore diameter between 0.1 µm and 3 µm.

16. Device in accordance with one or more of Claims 1 to 15, **characterized in that** the membrane has an essentially constant average pore diameter along at least 80% of its extent in the direction of its extent between the channel system and the at least one cavity (2).

17. Device in accordance with Claim 16, **characterized in that** the membrane has a layer on the side facing the channel system that has a smaller average pore diameter than the adjacent region of the membrane in the direction of the at least one cavity (2).

18. Device in accordance with Claim 17, **characterized in that** the layer is between 1 µm and 5 µm thick.

19. Device in accordance with one or more of Claims 17 or 18, **characterized in that** the average pore diameter in the layer is smaller by a factor of 5 to 50 than that in the adjacent region.

20. Device in accordance with one or more of Claims 1 to 19, **characterized in that** the membrane has an average porosity between 50 and 90% by volume.

21. Device in accordance with one or more of Claims 1 to 20, **characterized in that** the membrane has a BET surface between 2 and 300 m² per cm³ of membrane volume.

22. Device in accordance with Claim 21, **characterized in that** the membrane has a BET surface between 4 and 30 m² per cm³ of membrane volume.

23. Device in accordance with one or more of Claims 1 to 22, **characterized in that** the membrane is a hollow-fiber membrane and the wall (3) of the hollow-fiber membrane is the wall of the at least one cavity (2) and the hollow-fiber membrane is closed at least at one end.

24. Device in accordance with Claim 23, **characterized in that** the hollow-fiber membranes of a group of treatment elements (11) lying side by side are kept apart with textile threads (12).

25. Device in accordance with Claim 24, **characterized in that** the hollow-fiber membranes of a group are bound into at least one hollow-fiber mat by means of the textile threads (12).

26. Device in accordance with Claim 25, **characterized in that** the at least one hollow-fiber mat is a woven mat.

27. Device in accordance with Claim 25, **characterized in that** the at least one hollow-fiber mat is a knitted mat.

28. Device in accordance with Claim 25, **characterized in that** the at least one hollow-fiber mat is a woven tape.

29. Device in accordance with one or more of Claims 25 to 28, **characterized in that** the at least one hollow-fiber mat is spirally wound around an axis parallel to the direction of the extent of the channel system between the inlet arrangement (24) and the outlet arrangement (28).

30. Device in accordance with one or more of Claims 25 to 28, **characterized in that** at least two hollow-fiber mats are laid on top of each other and spirally wound around an axis parallel to the direction of the extent of the channel system between the inlet arrangement (24) and the outlet arrangement (28) and the hollow-fiber mats are laid on top of each other in such a way that the hollow fibers of the hollow-fiber mats laid on top of each other are brought into a criss-cross arrangement.

31. Device in accordance with one or more of Claims 1 to 22, **characterized in that** the at least one treatment element is formed from at least one flat membrane.

32. Device in accordance with Claim 31, **characterized in that** the at least one treatment element is formed from a flat membrane folded in a U-shape, wherein the fold edge formed by folding is arranged in the direction of the inlet arrangement (24) and wherein the arms (15) (16) of the flat membrane folded in a U-shape thus produced are kept apart in order to form the at least one cavity (2).

33. Device in accordance with Claim 31, **characterized in that** the at least one treatment element is formed from two flat membranes parallel to each other and kept apart from each other, wherein the two flat membranes are joined together positively at least at the edge they have facing in the direction of the inlet arrangement (24).

34. Device in accordance with one or more of Claims 31 to 33, **characterized in that** the at least one flat membrane is spirally wound around an axis parallel to the direction of the extent between the inlet arrangement (24) and the outlet arrangement (28) in such a manner that the closed edge faces in the direction of the inlet arrangement (24), wherein the laps are kept apart by spacers and by this means channels are formed between the laps, which are part of the channel system.

35. Device in accordance with one or more of Claims 1 to 34, **characterized in that** the membrane consists of polytetrafluoroethylene or polyvinylidene fluoride or modifications, blends, mixtures, or copolymers obtained from them.

36. Device in accordance with one or more of Claims 1 to 35, **characterized in that** the groups with a substance-specific action are ligands for affinic separation of ligates from the liquid to be treated.

37. Device in accordance with Claim 36, **characterized in that** the ligands are coupled to the membrane via molecules of long-chain linear polymers, whereby the molecules of the long-chain linear polymers carry a plurality of ligands.

38. Device in accordance with one or more of Claims 1 to 35, **characterized in that** the groups with a substance-specific action are enzymes.

39. Device in accordance with one or more of Claims 1 to 35, **characterized in that** the groups with a substance-specific action are catalysts.

40. Process for the substance-specific treatment of a fluid by interaction with target substances contained in the fluid to be treated and using a device in accordance with one or more of Claims 1 to 35, containing at least one treatment element that is at least partially formed from at least one semipermeable membrane with a porous structure, wherein the membrane has at least a first surface defining its exterior and at least a second surface defining its interior, comprising at least the steps:
a) Feeding the fluid to be treated into the casing,
b) Causing the same fluid to flow through the casing, wherein the fluid to be treated is caused to flow along the exterior of the membrane as a primary stream but not along its interior, in such a way that a part of this primary stream flows as a secondary stream into the membrane via the exterior, through the membrane, wherein the substance-specific treatment of the fluid takes place on the part of the fluid to be treated which forms the secondary stream, and then flows out through the interior of the membrane,
c) Removal of the treated fluid from the casing,
wherein the primary stream flows essentially freely along the exterior of the membrane over its entire extent and the secondary stream having passed through the membrane is reunited after the substance-specific treatment with the primary stream flowing along the exterior of the membrane.

41. Process in accordance with Claim 40, **characterized in that** the fluid to be treated is a suspension.

42. Process in accordance with one or more of Claims 40 or 41, **characterized in that** several treatment elements or groups of treatment elements are arranged in the device as stages spatially arranged one behind the other in the direction of the flow through the casing, and the fluid stream containing the primary stream and secondary stream is mixed between the individual stages.

43. Process in accordance with one or more of Claims 40 to 42, **characterized in that** several casings with treatment elements arranged in them are arranged one behind the other.

44. Process in accordance with one or more of Claims 40 to 43, **characterized in that** the fluid to be treated is recirculated.

45. Process in accordance with one or more of Claims 40 to 44 for the purification/separation of ligates from a liquid containing ligates by means of affinity, **characterized in that** a membrane is selected on and/or in which ligands for the aforementioned ligates are immobilized.

46. Process in accordance with one or more of Claims 40 to 44 for the enzymatic treatment of a liquid, **characterized in that** a membrane is selected on and/or in which enzymes are immobilized.

47. Process in accordance with one or more of Claims 40 to 44 for the catalytic treatment of fluids, **characterized in that** a membrane is used on and/or in which catalysts are immobilized.

## Revendications

1. Dispositif pour le traitement sélectif d'un fluide par interaction avec des substances cibles contenues dans le fluide à traiter, comprenant
a) un corps (25),
b) un dispositif d'entrée (24), pour l'introduction du fluide à traiter (23) dans le corps (25),
c) un dispositif de sortie (28), pour l'évacuation du fluide traité (27) hors du corps (25),
d) au moins un élément de traitement, pour le traitement sélectif du fluide, avec une extrémité tournée vers le dispositif d'entrée et une extrémité tournée vers le dispositif de sortie et une paroi (3) qui est au moins partiellement formée d'au moins une membrane semi-perméable avec une structure poreuse,
l'élément de traitement, au nombre d'au moins un, présentant une cavité (2) formée par la paroi (3) de l'élément de traitement, la cavité étant fermée ou tout au plus ouverte d'un côté, en direction du dispositif de sortie (28), l'élément de traitement, au nombre d'au moins un, étant disposé à l'intérieur du corps (25) de manière telle que soit formé, entre le dispositif d'entrée (24) et le dispositif de sortie (28), un système de canaux continu, qui est parcouru par le fluide à traiter, est en contact et entoure l'élément de traitement, au nombre d'au moins un, et enveloppe substantiellement l'élément de traitement, au nombre d'au moins un, au moins au niveau de son extrémité tournée vers le dispositif d'entrée et de son extrémité tournée vers le dispositif de sortie, des groupes à action sélective étant fixés sur et/ou dans la membrane.

2. Dispositif selon la revendication 1, dans lequel au moins une cavité (2) présente une ouverture (8) en direction du dispositif de sortie (28), qui débouche dans le système de canaux.

3. Dispositif selon une ou plusieurs des revendications 1 ou 2, **caractérisé en ce qu'**au moins une cavité (2) présente un rapport L/D de sa dimension L dans la direction d'extension du système de canaux entre le dispositif d'entrée (24) et le dispositif de sortie (28) au diamètre hydraulique D de sa section droite, qui est compris entre 2 et 4000.

4. Dispositif selon une ou plusieurs des revendications 1 à 3, **caractérisé en ce que** l'élément de traitement, au nombre d'au moins un, présente un rapport V_{w}/V_{b} du volume de sa paroi (3) V_{w} au volume de l'élément de traitement V_{b} composé du volume de la paroi (3) V_{w} et du volume Vₕ de la cavité (2), au nombre d'au moins une, compris entre 0,5 et 0,98.

5. Dispositif selon une ou plusieurs des revendications 1 à 4, **caractérisé en ce que** les parois (3) de chacun des éléments de traitement présentent une épaisseur essentiellement constante.

6. Dispositif selon une ou plusieurs des revendications 1 à 5, **caractérisé par** au moins un groupe de plusieurs éléments de traitement, qui sont disposés les uns à côté des autres, essentiellement transversalement à la direction d'extension du système de canaux entre le dispositif d'entrée (24) et le dispositif de sortie (28).

7. Dispositif selon la revendication 6, **caractérisé en ce que** les éléments de traitement regroupés en au moins un groupe sont maintenus écartés les uns des autres par des éléments d'espacement.

8. Dispositif selon la revendication 7, **caractérisé en ce que** les éléments de traitement disposés dans le corps (25) et voisins de la paroi interne du corps (25) présentent une distance par rapport à la paroi interne du corps, qui est inférieure ou égale à la distance entre les éléments de traitement.

9. Dispositif selon une ou plusieurs des revendications 1 à 8, **caractérisé en ce que** plusieurs éléments de traitement ou groupes d'éléments de traitement sont disposés les uns derrière les autres, en tant qu'étages (22), dans le corps (25), dans la direction d'extension du système de canaux, entre le dispositif d'entrée (24) et le dispositif de sortie (28).

10. Dispositif selon la revendication 9, **caractérisé en ce que** le nombre d'étages est compris entre 1 et 1000.

11. Dispositif selon la revendication 10, **caractérisé en ce que** le nombre d'étages est compris entre 2 et 100.

12. Dispositif selon une ou plusieurs des revendications 1 à 11, **caractérisé en ce que** le rapport V_{B}/V_{G} du volume total V_{B} de tous les éléments de traitement, formé des volumes V_{b} des différents éléments de traitement, au volume V_{G} du corps (25) vide est compris entre 0,4 et 0,95.

13. Dispositif selon la revendication 12, **caractérisé en ce que** le rapport est compris entre 0,55 et 0,75.

14. Dispositif selon une ou plusieurs des revendications 1 à 13, **caractérisé en ce que** la membrane présente un diamètre moyen de pore compris entre 0,005 µm et 5 µm.

15. Dispositif selon la revendication 14, **caractérisé en ce que** la membrane présente un diamètre moyen de pore compris entre 0,1 µm et 3 µm.

16. Dispositif selon une ou plusieurs des revendications 1 à 15, **caractérisé en ce que** la membrane, dans la direction d'extension entre le système de canaux et la cavité (2), au nombre d'au moins une, présente un diamètre moyen de pores essentiellement constant sur au moins 80% de la longueur.

17. Dispositif selon la revendication 16, **caractérisé en ce que** la membrane, sur sa face tournée vers le système de canaux, est pourvue d'une couche, qui présente un diamètre moyen de pores plus faible que la partie de la membrane voisine en direction de la cavité (2), au nombre d'au moins une.

18. Dispositif selon la revendication 17, **caractérisé en ce que** la couche a une épaisseur comprise entre 1 µm et 5 µm.

19. Dispositif selon une ou plusieurs des revendications 17 ou 18, **caractérisé en ce que** le diamètre moyen de pores dans la couche est inférieur d'un facteur de 5 à 50 au diamètre moyen de pores dans la région voisine.

20. Dispositif selon une ou plusieurs des revendications 1 à 19, **caractérisé en ce que** la membrane présente une porosité moyenne comprise entre 50% et 90% du volume.

21. Dispositif selon une ou plusieurs des revendications 1 à 20, **caractérisé en ce que** la membrane présente une surface BET comprise entre 2 et 300 m² par cm³ de volume de membrane.

22. Dispositif selon la revendication 21, **caractérisé en ce que** la membrane présente une surface BET comprise entre 4 et 30 m² par cm³ de volume de membrane.

23. Dispositif selon une ou plusieurs des revendications 1 à 22, **caractérisé en ce que** la membrane est une membrane à fibres creuses, **en ce que** la paroi (3) de la membrane à fibres creuses est la paroi de la cavité (2), au nombre d'au moins une, et **en ce que** la membrane à fibres creuses est fermée à une extrémité au moins.

24. Dispositif selon la revendication 23, **caractérisé en ce que** les membranes à fibres creuses d'un groupe d'éléments de traitement (11) disposés les uns à côté des autres sont maintenues écartées par des fils textiles (12).

25. Dispositif selon la revendication 24, **caractérisé en ce que** les membranes à fibres creuses d'un groupe d'éléments sont liées par les fils textiles (12) pour former au moins un mat de fibres creuses.

26. Dispositif selon la revendication 25, **caractérisé en ce que** le mat de fibres creuses, au nombre d'au moins un, est un mat tissé.

27. Dispositif selon la revendication 25, **caractérisé en ce que** le mat de fibres creuses, au nombre d'au moins un, est un mat tricoté.

28. Dispositif selon la revendication 25, **caractérisé en ce que** le mat de fibres creuses, au nombre d'au moins un, est une bande tissée.

29. Dispositif selon une ou plusieurs des revendications 25 à 28, **caractérisé en ce que** le mat de fibres creuses est enroulé en spirale autour d'un axe parallèle à la direction d'extension du système de canaux entre le dispositif d'entrée (24) et le dispositif de sortie (28).

30. Dispositif selon une ou plusieurs des revendications 25 à 28, **caractérisé en ce qu'**au moins deux mats de fibres creuses sont disposés l'un sur l'autre et enroulés en spirale autour d'un axe parallèle à la direction d'extension du système de canaux entre le dispositif d'entrée (24) et le dispositif de sortie (28), et **en ce que** les mats de fibres creuses sont disposés l'un sur l'autre de telle sorte que les fibres creuses des mats de fibres creuses superposés soient croisées.

31. Dispositif selon une ou plusieurs des revendications 1 à 22, **caractérisé en ce que** l'élément de traitement, au nombre d'au moins un, est formé d'au moins une membrane plane.

32. Dispositif selon la revendication 31, **caractérisé en ce que** l'élément de traitement, au nombre d'au moins un, est formé d'une membrane plane pliée en U, le bord de pliage résultant du pliage étant disposé en direction du dispositif d'entrée (24) et les ailes (15, 16) ainsi formées de la membrane plane pliée en U étant maintenues écartées pour former la cavité (2), au nombre d'au moins une.

33. Dispositif selon la revendication 31, **caractérisé en ce que** l'élément de traitement, au nombre d'au moins un, est formé de deux membranes planes mutuellement parallèles et disposées à distance l'une de l'autre, les deux membranes planes étant liées l'une à l'autre par obstacle au moins au niveau de leur bord tourné vers le dispositif d'entrée (24).

34. Dispositif selon une ou plusieurs des revendications 31 à 33, **caractérisé en ce que** la membrane plane, au nombre d'au moins une, est enroulée en spirale autour d'un axe parallèle à la direction d'extension du système de canaux entre le dispositif d'entrée (24) et le dispositif de sortie (28), de telle sorte que le bord fermé soit tourné vers le dispositif d'entrée (24), les couches enroulées étant maintenues écartées par des éléments d'espacement, et des canaux qui font partie du système de canaux étant formés entre les couches enroulées.

35. Dispositif selon une ou plusieurs des revendications 1 à 34, **caractérisé en ce que** la membrane est polytétrafluoréthylène ou en polyfluorure de vinyle ou en des variantes de ces produits, des blends, des mélanges ou des copolymères.

36. Dispositif selon une ou plusieurs des revendications 1 à 35, **caractérisé en ce que** les groupes à action sélective sont des ligants pour la séparation affine de ligats du liquide à traiter.

37. Dispositif selon la revendication 36, **caractérisé en ce que** les ligants sont couplés à la membrane par des molécules de polymères linéaires à longue chaîne, les molécules des polymères linéaires à longue chaîne portant plusieurs ligants.

38. Dispositif selon une ou plusieurs des revendications 1 à 35, **caractérisé en ce que** les groupes à action sélective sont des enzymes.

39. Dispositif selon une ou plusieurs des revendications 1 à 35, **caractérisé en ce que** les groupes à action sélective sont des catalyseurs.

40. Procédé pour le traitement sélectif d'un fluide par interaction avec des substances cibles contenues dans le fluide, faisant usage d'un dispositif selon une ou plusieurs des revendications 1 à 35, comportant au moins un élément de traitement, qui est au moins partiellement formé d'au moins une membrane semi-perméable à structure poreuse, la membrane présentant au moins une première surface formant la face extérieure et au moins une deuxième surface formant la face intérieure, ledit procédé comprenant au moins les étapes suivantes :
a) introduction du fluide à traiter dans le corps,
b) écoulement dudit fluide à travers le corps, le fluide à traiter arrivant en tant que flux primaire sur la face extérieure de la membrane et pas sur sa face intérieure, de telle sorte qu'une partie dudit flux primaire entre en tant que flux secondaire dans la membrane, via la face extérieure, s'écoule à travers la membrane, le traitement sélectif du fluide étant opéré sur la fraction du fluide à traiter qui constitue le flux secondaire, et enfin sorte de la membrane à travers la face intérieure,
c) évacuation du fluide traité hors du corps, la membrane, sur toute sa longueur, étant entourée librement par le flux primaire au niveau de sa face extérieure et le flux secondaire qui a traversé la membrane, après le traitement sélectif, étant ramené dans le flux primaire sur la face extérieure de la membrane.

41. Procédé selon la revendication 40, **caractérisé en ce que** le fluide à traiter est une suspension.

42. Procédé selon une ou plusieurs des revendications 40 ou 41, **caractérisé en ce que** dans le dispositif, plusieurs éléments de traitement ou groupes d'éléments de traitement sont disposés l'un derrière l'autre, en tant qu'étages, dans la direction d'écoulement à travers le corps, et **en ce qu'**entre les différents étages, le flux de fluide comprenant le flux primaire et le flux secondaire est mélangé.

43. Procédé selon une ou plusieurs des revendications 40 à 42, **caractérisé en ce que** plusieurs corps, avec des éléments de traitement disposés à l'intérieur de ceux-ci, sont connectés l'un derrière l'autre.

44. Procédé selon une ou plusieurs des revendications 40 à 43, **caractérisé en ce que** le fluide à traiter est recyclé.

45. Procédé selon une ou plusieurs des revendications 40 à 44 pour l'épuration/séparation par affinité de ligats dans un liquide contenant des ligats, **caractérisé en ce qu'**on utilise une membrane sur laquelle et/ou dans laquelle des ligands pour lesdits ligats sont fixés.

46. Procédé selon une ou plusieurs des revendications 40 à 44 pour le traitement par enzymes d'un liquide contenant, **caractérisé en ce qu'**on utilise une membrane sur laquelle et/ou dans laquelle des enzymes sont fixés.

47. Procédé selon une ou plusieurs des revendications 40 à 44 pour le traitement catalytique de fluides, **caractérisé en ce qu'**on utilise une membrane sur laquelle et/ou dans laquelle des catalyseurs sont fixés.
